(19) Europäisches Patentamt
European Patent Office
Office européen des brevets

(11) EP 2 022 850 B1

(12) **EUROPEAN PATENT SPECIFICATION**

(45) Date of publication and mention of the grant of the patent:
**31.08.2011 Bulletin 2011/35**

(21) Application number: **07744128.5**

(22) Date of filing: **25.05.2007**

(51) Int Cl.:
*C12N 9/04* (2006.01) *C12N 1/14* (2006.01)
*C12Q 1/32* (2006.01) *A61K 47/48* (2006.01)
*G01N 21/75* (2006.01)

(86) International application number:
**PCT/JP2007/060694**

(87) International publication number:
**WO 2007/139013 (06.12.2007 Gazette 2007/49)**

(54) **FLAVIN ADENINE DINUCLEOTIDE-BINDING GLUCOSE DEHYDROGENASE**

FLAVIN-ADENIN-DINUKLEOTID BINDENDE GLUCOSE-DEHYDROGENASE

GLUCOSE DÉSHYDROGÉNASE LIANT LE FLAVINE ADÉNINE DINUCLÉOTIDE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.05.2006 JP 2006148667**

(43) Date of publication of application:
**11.02.2009 Bulletin 2009/07**

(73) Proprietor: **Amano Enzyme Inc.**
**Nagoya-shi,**
**Aichi 460-0003 (JP)**

(72) Inventors:
- **TANAKA, Noriaki**
  **Kakamigahara-shi, Gifu 5090109 (JP)**
- **YUUKI, Kensuke**
  **Kakamigahara-shi, Gifu 5090109 (JP)**

(74) Representative: **Müller Fottner Steinecke**
**Rechtsanwälte Patentanwälte**
**Technologiezentrum Jülich**
**Karl-Heinz-Beckurts-Strasse 13**
**52428 Jülich (DE)**

(56) References cited:
**EP-A- 0 094 161** **WO-A-03/012071**
**WO-A-2007/116710** **WO-A1-2004/058958**

- **SINOHARA H: "EFFECT OF O-PHENANTHROLINE ON THE INDUCTION OF GLUCOSE DEHYDROGENASE IN ASPERGILLUS ORYZAE" BIOCHEMICAL AND BIOPHYSICAL RESEARCH COMMUNICATIONS, ACADEMIC PRESS INC. ORLANDO, FL, US, vol. 33, no. 2, 1 January 1968 (1968-01-01), pages 197-202, XP009063975 ISSN: 0006-291X**
- **KOJIMA S. ET AL.: 'Fundamental study for an oxygen-insensitive amperometric glucose sensor using a novel glucose dehydrogenase' CHEMICAL SENSORS vol. 20, no. SUPPL. B, 2004, pages 768 - 769, XP003004574**
- **TSUJIMURA S. ET AL.: 'Novel FAD-dependent glucose dehydrogenase for a dioxygen-insensitive glucose biosensor' BIOSCI. BIOTECHNOL. BIOCHEM. vol. 70, no. 3, March 2006, pages 654 - 659, XP003004577**
- **BAK T.-G.: 'Studies on glucose dehydrogenase of Aspergillus oryzae. II. Purification and physical and chemical properties' BIOCHIM. BIOPHYS. ACTA vol. 139, no. 2, 1967, pages 277 - 293, XP009067679**
- **BAK T.-G.: 'Studies on glucose dehydrogenase of Aspergillus oryzae. 3. General enzymatic properties' BIOCHIM. BIOPHYS. ACTA vol. 146, no. 2, 1967, pages 317 - 327, XP002373580**
- **DATABASE GENPEPT [Online] MACHIDA M. ET AL., XP003019816 Database accession no. (Q2USF2) & NATURE vol. 438, no. 7071, 2005, pages 1157 - 1161**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 022 850 B1

## Description

TECHNICAL FIELD

The present invention relates to a coenzyme-binding glucose dehydrogenase and the application thereof. More particularly, the present invention relates to a glucose dehydrogenase whose coenzyme is flavin adenine dinucleotide, a fungus producing the enzyme, a method for manufacturing the enzyme, and a method for measuring glucose by using the enzyme.

BACKGROUND ART

Recently, a simplified blood sugar self-measuring device using an electrochemical biosensor has been widely used. The biosensor includes an electrode and an enzyme reaction layer on an insulating substrate. An example of the enzyme to be used herein includes glucose dehydrogenase (GDH), glucose oxidase (GO), and the like. The method using GO is susceptible to dissolved oxygen in a sample to be measured. Thus, the dissolved oxygen may affect the measurement results.

Meanwhile, as an enzyme that is not susceptible to dissolved oxygen and acts on glucose in the absence of NAD (P), GDH (PQQ-GDH) whose coenzyme is pyrrolo-quinoline quinone (PQQ) is known (see, for example, patent documents 1 to 3). However, PQQ-GDH has the following problems: (1) PQQ is easily dissociated from enzyme; (2) selectivity with respect to glucose is low; and (3) extraction and isolation operation are difficult because it is present on the membrane fraction.

Recently, a case, in which a patient using a simplified blood sugar self-measuring device in a hospital develops hypoglycemia and the like, has been reported. As a result of examination and analysis conducted thereafter, the cause of such case is determined that PQQ-GDH reacts with maltose contained as a component of an infusion solution, exhibiting a blood sugar value higher than the actual value (Pharmaceuticals and Medical Devices Safety Information No.206, October 2004, Pharmaceutical and Food Safety Bureau, Ministry of Health, Labor and Welfare). Under such circumstances, the development of an enzyme for measuring blood sugar, which specifically acts on glucose and has a low activity with respect to maltose, has been demanded.
Note here that besides PQQ-GDH, as an enzyme that is not susceptible to dissolved oxygen and acts on glucose in the absence of NAD (P), glucose dehydrogenase whose coenzyme is flavin adenine dinucleotide is known (also referred to as "FAD-GDH" in this specification) is known. To date, FAD-GDH has been obtained from Aspergillus oryzae (non-patent documents 1 - 4) and Aspergillus terreus (patent document 4), respectively.

[Patent document 1] Japanese Patent Application Unexamined Publication No. 2000-350588
[Patent document 2] Japanese Patent Application Unexamined Publication No. 2001-197888
[Patent document 3] Japanese Patent Application Unexamined Publication No. 2001-346587
[Patent document 4] WO 2004/058958
[Non-patent document 1] Studies on the glucose dehydrogenase of Aspergillus oryzae. I. Induction of its synthesis by p-benzoquinone and hydroquinone, T.C. Bak, and R. Sato, Biochim. Biophys. Acta, 139, 265-276 (1967).
[Non-patent document 2] Studies on the glucose dehydrogenase of Aspergillus oryzae. II. Purification and physical and chemical properties, T.C. Bak, Biochim. Biophys. Acta, 139, 277-293 (1967).
[Non-patent document 3] Studies on the glucose dehydrogenase of Aspergillus oryzae. III. General enzymatic properties, T.C. Bak, Biochim. Biophys. Acta, 146, 317-327 (1967).
[Non-patent document 4] Studies on the glucose dehydrogenase of Aspergillus oryzae. IV Histidyl residue as an active site, T.C. Bak, and R. Sato, Biochim. Biophys. Acta, 146, 328-335 (1967).

[DISCLOSURE OF THE INVENTION]

[Problems to be Solved by the Invention]

Under the background mentioned above, it is an object of the present invention to provide a new enzyme that allows accurate measurement of the amount of glucose, and a fungus producing the enzyme, as well as the application thereof.

[Means to Solve the Problems]

In order to achieve the above-mentioned object, the present inventors have paid attention to glucose dehydrogenase (FAD-GDH) whose coenzyme is flavin adenine dinucleotide. Then, they have carried out screening of wide variety of microorganisms, and found a strain having an excellent productivity of FAD-GDH, which has been identified to be Aspergillus oryzae. Furthermore, they have succeeded in purifying FAD-GDH produced by the strain and determining the various properties thereof. From the determined properties, it is determined that the FAD-GDH is a new enzyme. Furthermore, it is shown that the FAD-GDH is excellent in selectivity with respect to glucose, is less susceptible to the dissolved oxygen existing in the reaction system, and allows accurate measurement of the amount of glucose in a sample. On the other hand, it is confirmed that other strain (Aspergillus oryzae) produces an enzyme that is equal to FAD-GDH.
The present inventors have carried out further investigation and identifies the amino acid sequence of FAD-GDH possessed by the strain and the base sequence of a gene encoding the amino acid sequence.
The present invention has been made based on the findings or results mentioned above, and it provides flavin adenine dinucleodde-binding glucose dehydrogenase shown below, and the like.

[1] Flavin adenine dinucleotide-binding glucose dehydrogenase consisting of the amino acid sequence set forth in SEQ ID NO: 20 and comprising the following properties:

(1) action: catalyzing a reaction of oxidizing a hydroxyl group of glucose in a presence of an electron acceptor to yield glucono-δ-lactone;
(2) molecular weight: about 100 kDa when measured by SDS-polyacrylamide electrophoresis and about 400 kDa when measured by gel filtration chromatography; and
(3) substrate specificity: having low reactivity with respect to maltose, D-fructose, D-mannose and D-galactose. wherein the reactivity with respect to maltose is 5% or less when the reactivity with respect to D-glucose is assumed to be 100%; wherein the reactivity with respect to D-galactose is 5% or less when the reactivity with respect to D-glucose is assumed to be 100%; wherein the reactivities with respect to D-fcuctose and D-mannose are 5% or less when the reactivity with respect to D-glucose is assumed to be 100%.

[2] The flavin adenine dinucleotide-binding glucose dehydrogenase according to [1], further comprising the following properties:

(4) optimum pH: about 7;
(5) optimum temperature: about 60°C;
(6) pH for stability: stable in the range from pH 3.0 to pH 7.0; and
(7) temperature for stability: stable at 40°C or less.

[3] The flavin adenine dinucleotide-binding glucose dehydrogenase according to [2], further comprising the following property:

(8) Km value: about 8 mM with respect to D-glucose.

[4] The flavin adenine dinucleotide-binding glucose dehydrogenase according to any one of [1] to [3], which is derived from Aspergillus oryzae.
5. Aspergillus oryzae BB-56 deposited under the accession number of NITE BP-236, which has an ability to produce flavin adenine dinucleotide-binding glucose dehydrogenase.
6. A flavin adenine dinucleotide-binding glucose dehydrogenase gene
7. A vector containing the gene according to [6].
8. A transformant in which the gene according to [6] is introduced.
9. A method for manufacturing flavin adenine dinucleotide-binding glucose dehydrogenase, the method comprising the following steps (1) and (2) or steps (i) and (ii):

(1) culturing Aspergillus oryzae having an ability to produce the flavin adenine dinucleotide-binding glucose dehydrogenase according to [4] ;
(2) recovering the flavin adenine dinucleotide-binding glucose dehydrogenase from a culture solution end/or a fungus body after culturing;

(i) culturing a transformant according to [8] under a condition in which protein encoded by the gene is produced; and
(ii) recovering the produced protein.

[10] A method for measuring glucose, wherein glucose in a sample is measured by using the flavin adenine dinucleotide-binding glucose dehydrogenase according to any one of [1] to [4].

[11] A reagent for measuring glucose, comprising the flavin adenine dinucleotide-binding glucose dehydrogenase according to any one of [1] to [4].

12. A kit for measuring glucose comprising the reagent for measuring glucose according to [11].

[BRIEF DESCRIPTION OF THE DRAWINGS]

Fig. 1 shows SDS-PAGE using purified FAD-GDH derived from Aspergillus oryzae BB-56. Lanes at both ends show molecular weight markers. Phosphorylase b (97 kDa), albumin (66 kDa), ovalbumin (45 kDa), carbonic anhydrase (30 kDa), trypsin inhibitor (20.1 kDa), and α-lactalbumin (14.4 kDa) are shown from the upper side in this order.

Fig. 2 shows measurement of a molecular weight by HPLC of purified FAD-GDH derived from Aspergillus oryzae BB-56. (A) shows molecular weight markers, showing glutamic acid dehydrogenase (290 kDa, 11.508 min), lactate dehydrogenase (142 kDa, 13.012 min), enolase (67 kDa, 14.643 min), myokinase (32 kDa, 15.321 min), and cytochrome C (12.4 kDa, 20.303 min). (B) shows purified FAD-GDH.

Fig. 3 shows comparison of a molecular weight of FAD-GDH derived from Aspergillus oryzae. (A) molecular weight markers showing glutamic acid dehydrogenase (GLDH, 290 kDa), lactate dehydrogenase (LDH, 142 kDa), myokinase (32 kDa) in the order elution is earlier. (B), (C), (D), (E), (F), (G), and (H) use culture filtrate derived from A. oryzae BB-56, IAM2603, IAM2628, IAM2683, IAM2736, IAM2706 and NBRC30113, respectively.

Fig. 4 is a graph showing an absorption spectrum in 400-800 nm of the purified FAD-GDH derived from Aspergillus oryzae BB-56.

Fig. 5 is a graph showing an optimum pH of purified FAD-GDH derived from Aspergillus oryzae BB-56.

Fig. 6 is a graph showing an optimum temperature of purified FAD-GDH derived from Aspergillus oryzae BB-56.

Fig. 7 is a graph showing a pH for stability of purified FAD-GDH derived from Aspergillus oryzae BB-56.

Fig. 8 is a graph showing a temperature for stability of purified FAD-GDH derived from Aspergillus oryzae BB-56.

Fig. 9 is a graph showing measured isoelectric point of purified FAD-GDH derived from Aspergillus oryzae BB-56 by glycerol density gradient isoelectric point electrophoresis.

Fig. 10 shows Lineweaver-Burk plot of purified FAD-GDH derived from Aspergillus oryzae BB-56 with respect to glucose.

Fig. 11 is a graph showing the measurement of the content of glucose in purified FAD-GDH derived from Aspergillus oryzae BB-56.

Fig. 12 is a graph showing the measurement of the glucose level using purified FAD-GDH derived from Aspergillus oryzae BB-56.

[BEST MODE OF CARRYING OUT THE INVENTION]

(Terms)

In the present invention, the "DNA encoding protein" refers to DNA producing a protein when it is expressed, that is, DNA having a base sequence corresponding to the amino acid sequence of the protein. Therefore, degeneracy of codon is also considered.

In the present invention, the term "isolated" can be used exchangeably with "purified." The term "isolated" used regarding to enzyme (FAD-GDH) of the present invention denotes that when the enzyme of the present invention is derived from a natural material, the enzyme exists in a state in which it does not substantially include a component other than the enzyme in the natural material (in particular, the enzyme does not substantially contain a foreign protein). Specifically, for example, the content of the contaminated protein in the isolated enzyme of the present invention is, for example, less than about 20%, preferably less than about 10%, further preferably less than about 5%, and yet further preferably less than about 1% with respect to the total amount on a weight basis. On the other hand, when the enzyme of the present invention is prepared by genetically engineering technique, the term "isolated" denotes a state in which the enzyme does not substantially contain the other component derived from a host cell to be used and a culture solution and the like. Specifically, for example, in the isolated enzyme of the present invention, the content of contaminated components is less than about 20%, preferably less than about 10%, further preferably less than about 5%, and yet further preferably less than about 1% with respect to the total amount on a weight basis. Unless otherwise noted, when merely the term "flavin adenine dinucleotide-binding glucose dehydrogenase" in the present invention means "flavin adenine dinucleotide-binding glucose dehydrogenase in the isolated state." The same is true to the terms "FAD-GDH" and "enzyme" to be used instead of the term "flavin adenine dinucleotide-binding glucose dehydrogenase."

The term "isolated" to be used for DNA typically means a state in which the DNA is separated from the other nucleic acid coexisting in a natural state when the DNA is in an originally natural state. However, it may contain a part of other nucleic acid component such as flanking nucleic acid sequence in a natural state (for example, sequence in the promoter region, terminator sequence, or the like). For example, it is preferable that the "isolated" state in genome DNA does not substantially contain other DNA components coexisting in a natural state. On the other hand, it is preferable that the "isolated" state of DNA such as cDNA molecule prepared by genetically engineering technique does not substantially contain a cell component, a culture solution, and the like. Similarly, it is preferable that the "isolated" state of DNA prepared by the chemical synthesis does not substantially contain a precursor (a raw material) such as dNTP or chemical substances to be used in the synthesizing process, and the like. Unless otherwise noted, when merely the term "DNA" in the present invention means isolated DNA.

(FAD-GDH and fungus producing the enzyme)

The first aspect of the present invention provides glucose dehydrogenase (FAD-GDH) whose coenzyme is flavin adenine dinucleotide, and fungus producing the same. FAD-GDH of the present invention (hereinafter, which is also referred to as "the enzyme") is characterized by having the following properties. Firstly, the enzyme catalyzes the following reaction, that is, a reaction of oxidizing a hydroxyl group of glucose in the presence of an electron acceptor to yield glucono-δ-lactone. Furthermore, the molecular weight of the enzyme is about 100 kDa when measured by SDS-polyacrylamide electrophoresis and about 400 kDa when measured by gel filtration chromatography. Note here that the FAD-GDH of the present invention consists of tetramer.

Meanwhile, the enzyme is excellent in the substrate specificity and acts on D-glucose selectivity. In detail, the FAD-GDH of the present invention has extremely low reactivity with respect to maltose and also low reactivity with respect to D-fructose, D-mannose, D-galactose, or the like. Specifically, the reactivity with respect to these substrates is 5% or less when the reactivity with respect to D-glucose is 100%. In the preferable exemplary embodiment of the present invention, the reactivity with respect to maltose is 1% or less or substantially zero. In the more preferable exemplary embodiment, the reactivities with respect to D-fructose and D-galactose are also 1% or less or substantially zero. The enzyme having such an excellent substrate specificity is preferable as an enzyme for accurately measuring the amount of glucose in a sample. That is to say, with the enzyme, even if the sample includes a foreign matter such as maltose, galactose, or the like, it is possible to accurately measure the amount of the intended glucose. Therefore, the enzyme is suitable for application in which it is expected or concerned that the sample may include such a foreign matter (typically, application of measuring the amount of glucose in blood). Furthermore, the enzyme is suitable for various applications including this application, that is, the enzyme has versatility. Note here that the reactivity and the substrate specificity can be measured and evaluated by the methods described in the below mentioned Examples (columns (1-2), (7-1) and (7-2-2)).

It is preferable that the enzyme is FAD-GDH derived from Aspergillus oryzae. The "FAD-GDH derived from Aspergillus oryzae" herein denotes FAD-GDH produced by microorganism (which may be wild strain or variant) classified in Aspergillus oryzae, or FAD-GDH obtained by genetically engineering technique by using FAD-GDH gene of Aspergillus oryzae (which may be wild strain or variant). Therefore, the recombinant produced by a host microorganism, into which the FAD-GDH gene obtained from Aspergillus oryzae (or gene obtained by modifying the gene) has introduced, also corresponds to "FAD-GDH derived from Aspergillus oryzae."

Aspergillus oryzae from which the enzyme is derived is referred to as a fungus producing the enzyme for the sake of convenience. An example of the fungus producing the enzyme can include Aspergillus oryzae BB-56, Aspergillus oryzae IAM2603, Aspergillus oryzae IAM2628, Aspergillus oryzae IAM2683, Aspergillus oryzae IAM2736, Aspergillus oryzae IAM2706, and Aspergillus oryzae NBRC30113 shown in the below-mentioned Examples. Among them, BB-56 strain is particularly preferable fungus producing the enzyme because it is excellent in the productivity of FAD-GDH. BB-56 strain is deposited with the following predetermined depositary agency and easily available.

The Institute of deposition: NITE (National Institute of Technology and Evaluation), Department of biotechnology, patent microorganism deposit center (Location: 2-5-8, Kazusakamatari, Kisarazu-city, Chiba, 292-0818, Japan)
Deposition date (reception date): May 17, 2006
Accession number: NITE BP-236

Note here that Aspergillus oryzae IAM2603, Aspergillus oryzae IAM2628, Aspergillus oryzae IAM2683, Aspergillus oryzae IAM2736, and Aspergillus oryzae IAM2706 are strains stored in IAM culture collection (Institute of Molecular and Cellular Biosciences, Cell Function Information Research Center, University of Tokyo), which can be available via predetermined procedure. Similarly, Aspergillus oryzae NBRC30113 is a strain stored in NBRC (National Institute of Technology and Evaluation, Department of Biotechnology, Department of Genetic Resource of Organisms), which can be available via predetermined procedure.

As shown in the following Examples, the present inventors have succeeded in preparing FAD-GDH having the above-mentioned properties (action, molecular weight, and substrate specificity) from Aspergillus oryzae BB-56. As a result of the detail investigation, it is clear that the obtained FAD-GDH further has the following properties.

(4) Optimum pH: about 7
(5) Optimum temperature : about 60°C
(6) pH for stability: stable at pH in the range from 3.0 to 7.0
(7) Temperature stability: stable at 40°C or less
(8) Km value: Km value with respect to D-glucose is about 8 mM
(9) Isoelectric point: about 6.4

Herein, the optimum pH is a value measured in, for example, Mcllvaine buffer as mentioned below in the Examples. The optimum temperature is also a value measured in, for example, PIPES-NaOH buffer (pH 6.5). Furthermore, the enzyme maintains 80% or more of the activity when it is treated under a certain pH condition at 37°C for 30 minutes, the enzyme can be "stable" in the pH condition. Similarly, when the activity of the enzyme is not substantially deteriorated (that is to say, about 100% of the activity is maintained) after it is treated under a certain temperature condition in a suitable buffer solution (for example, PIPES-NaOH buffer, pH 6.5) for 20 minutes, the enzyme can be "stable" in the temperature condition. The Km value and the isoelectric point can be measured and evaluated by the method described below in the Examples (columns 8 and 6).

As mentioned above, the details of the properties of the obtained enzyme have been clarified. As a result, it is determined that the enzyme has selectivity and affinity with respect to glucose, is excellent in stability and suitable for application and commercialization of, for example, a glucose sensor.
On the other hand, as a result that the details of the properties of the enzyme have been clarified, the enzyme is confirmed to be an enzyme that is utterly different from the coenzyme-binding enzymes reported in the past.
Note here that it is confirmed that the above-mentioned other strains, that is, Aspergillus oryzae IAM2603, Aspergillus oryzae IAM2628, Aspergillus oryzae IAM2683, Aspergillus oryzae IAM2736, Aspergillus oryzae IAM2706, and Aspergillus oryzae NBRC30113 produce FAD-GDH having the properties that are equal to FAD-GDH produced by Aspergillus oryzae BB-56.

As a result of the further investigation by the present inventors, the amino acid sequence of FAD-GDH possessed by Aspergillus oryzae BB-56 has been determined. Then, the enzyme can be characterized by being a protein having the amino acid sequence set forth in SEQ ID NO: 20. Herein, in general, when a part of the amino acid sequence of a certain protein is modified, the modified protein may sometimes have the equal function as the protein before modification. That is to say, the modification of the amino acid sequence does not have substantial effect on the function of a protein, so that the function of the protein may be often maintained before and after the modification. Thus, as another exemplary embodiment, the present application describes a protein having an amino acid sequence homologous to the amino acid sequence set forth in SEQ ID NO: 20 and the FAD-GDH activity (hereinafter, which is referred to as "homologous protein"). The "homologous amino acid sequence" herein denotes an amino acid sequence that is partly different from the amino acid sequence set forth in SEQ ID NO: 20 but that is not have a substantial effect on the function (herein, FAD-GDH activity) of the protein. The "partial difference in the amino acid sequence" typically denotes that mutation (change) occurs in an amino acid sequence due to deletion or substitution of one to several amino acids constituting the amino acid sequence, or addition or insertion of one to several amino acids constituting the amino acid sequence, or the combination thereof. The difference in the amino acid sequence is permitted as long as the FAD-GDH activity is maintained (more or less change in the activity is permitted). As long as this condition is satisfied, the position of difference in the amino acid sequence is not particularly limited and the difference may occur in a plurality of positions. The plurality herein signifies a numerical value corresponding to less than about 30%, preferably less than about 20%, further preferably less than about 10%, yet further preferably less than about 5%, and most preferably less than about 1% with respect to the entire amino acid. That is to say, homologous protein has, for example, about 70% or more, preferably about 80% or more, further preferably about 90% or more, yet further preferably about 95% or more and most preferably about 99% or more of similarly to the amino acid sequence set forth in SEQ ID NO: 20.

Preferably, homologous protein is obtained by allowing conservative amino acid substitution to be generated in an amino acid residue that is not essential to the FAD-GDH activity. Herein, "conservative amino acid substitution" denotes a substitution of an amino acid residue to an amino acid residue having a side chain of the same property. The amino acid residue is classified into some families according to its side chain, for example, the basic side chain (for example, lysin, arginine, histidine), the acidic side chain (for example, asparatic acid, glutamic acid), the uncharged polar side chain (for example, glycine, asparagine, glutamine, serine, threonine, tyrosine, cysteine), the nonpolar side chain (for example, alanine, valine, leucine, isoleucine, proline, phenyl alanine, methionine, tryptophane), β-branched side chain (for example, threonine, valine, isoleucine), and the aromatic side chain (for example, tyrosine, phenyl alanine, tryptophane, histidine). The conservative amino acid substitution is preferably carried out between the amino acid residues in the same family.

The identity (%) of two amino acid sequences or of two nucleic acid sequences (hereinafter, referred to as "two sequences" as a term including these sequences) can be determined by, for example, the following procedures. Firstly, the two sequences are aligned so that two sequences can be optimally compared with each other (e.g., gaps may be introduced in a first sequence so as to optimize alignment with the second sequence). When the molecule (amino acid residue or nucleotide) at the certain position of the first sequence is the same as the molecule at corresponding position of the second sequence, then the molecules are defined to be identical to each other at that position. The identity between the two sequences is a function of the number of identical positions that are common in the two sequences (i.e. identity (%) = number of identical positions / total number of positions x 100). Preferably, the number of gaps, and the length of each gap, which need to be introduced for optimizing the alignment of the two sequences are also considered.
The comparison of sequences and determination of identity between two sequences can be accomplished using a mathematical algorithm. A specific example of the mathematical algorithm that can be used for comparison between sequences includes a mathematical algorithm that is described in Karlin and Altschul (1990) Proc. Natl. Acad. Sci. USA 87:2264-68 and that was modified by Karlin and Altschul (1993) Proc. Natl. Acad. Sci. USA 90:5873-77. However, the mathematical algorithm is not particularly limited to this. Such an algorithm is incorporated into NBLAST program and XBLAST program (version 2.0). In order to obtain a nucleotide sequence being homologous to the nucleic acid molecule of the present invention, for example, with NBLAST program, BLAST nucleotide search may be carried out with score = 100 and wordlength = 12. In order to obtain an amino acid sequence being homologous to the polypeptide molecule of the present invention, for example, with XBLAST program, BLAST polypeptide search may be carried out with score = 50 and wordlength = 3. In order to obtain a gap alignment for comparison, Gapped BLAST described in Altschul et al. (1997) Amino Acids Research 25 (17): 3389-3402 can be used. In the case where BLAST and Gapped BLAST are used, default parameter of the corresponding program (for example, XBLAST and NBLAST) can be used. In detail, see http://www.ncbi.nlm.nih.gov. Another example of a mathematical algorithm that can be used for comparison of sequences includes an algorithm described in Myers and Miller (1988) Comput Appl Biosci. 4:11-17. Such an algorithm is incorporated into an ALIGN program that can be used in, for example, a GENESTREAM network server (IGH Montpellier, France) or an ISREC server. In the case where the ALIGN program is used for comparison of the amino acid sequences, for example, PAM120 residue mass table is used, and gap length penalty can be made to be 12 and gap penalty can be made to be 4.
The identity between two amino acid sequences can be determined using the GAP program in the GCG software package using either a Blossum 62 matrix or a PAM250 matrix with the gap weight of 12, 10, 8, 6, or 4 and the gap length weight of 2, 3, or 4. Furthermore, the homology between two nucleic acid sequences can be determined using the GAP program in the GCG software package (usable in http://www.gcg.com) with a gap weight of 50 and a gap length weight of 3.

The enzyme having the above-mentioned amino acid sequence can be prepared easily by a genetic engineering technique. For example, an appropriate host cell (for example, Escherichia coli) is transformed by using DNA encoding the enzyme, and recovering protein expressed in the transformant. The recovered protein is purified appropriately in accordance with the purposes. In the case where the enzyme is prepared as recombinant protein, various modifications can be carried out. For example, DNA encoding the enzyme and other appropriate DNA are inserted into the same vector and the vector is used for producing recombinant protein. Then, the enzyme including a recombinant protein in which other peptide or protein is binding can be obtained. Furthermore, modification such as addition of sugar chain and/or lipid or processing of N-terminus or C-terminus may be carried out. The above-mentioned modification enables extraction of recombinant protein, simplification of publication, addition of biological functions, or the like.

(DNA encoding FAD-GDH)

The second aspect of the present invention provides a gene encoding the enzyme, that is a new FAD-GDH gene. In one exemplary embodiment, the gene described in the present application includes DNA encoding the amino acid sequence set forth in SEQ ID NO: 20. Specific example of the embodiment of the present invention is DNA consisting of the base sequence set forth in SEQ ID NO: 19.

In general, when a part of DNA encoding a certain protein is modified, protein encoded by modified DNA may sometimes have the equal function as a protein before modification. That is to say, the modification of the DNA sequence does not have substantial effect on the function of the encoded protein, so that the function of the encoded protein is often maintained before and after the modification. Thus, as another exemplary embodiment, the present application describes DNA encoding a protein having a base sequence homologous to the base sequence set forth in SEQ ID NO: 19 and the FAD-GDH activity (hereinafter, which is referred to as "homologous DNA"). The "homologous DNA" herein denotes a base sequence that is partly different from the base sequence set forth in SEQ ID NO: 19 but that does not substantially affect the function of the protein encoded by the base sequence (herein, FAD-GDH activity) due to the partial difference.

Specific example of the homologous DNA includes DNA that hybridizes to the complementary base sequence of base sequence of SEQ ID NO.: 19 under stringent conditions. Herein, "stringent conditions," are referred to as conditions in which a so-called specific hybrid can be formed and a nonspecific hybrid cannot be formed. Such stringent conditions are known to person skilled in the art. Such stringent conditions can be set with reference to, for example, Molecular Cloning (Third Edition, Cold Spring Harbor Laboratory Press, New York) and Current protocols in molecular biology (edited by Frederick M. Ausubel et al., 1987). An example of the stringent conditions can include a condition in which a hybridization solution (50% formamide, 10 x SSC (0.15M NaCl. 15 mM sodium citrate, pH 7.0), 5 x Denhardt solution, 1% SDS, 10% dextran sulfate, and 10 μg/ml modified salmon sperm DNA, 50 mM phosphate buffer (pH 7.5)) is used and incubated at about 42°C to about 50°C, thereafter, 0.1 x SSC and 0.1% SDS are used and washed at about 65°C to about 70°C. Further preferable stringent conditions can include conditions in which, for example, a hybridization solution (50% formamide, 5 x SC (0.15M NaCl, 15 mM sodium citrate, pH 7.0), 1 x Denhardt solution, 1% SDS, 10% dextran sulfate, 10 μg/ml modified salmon sperm DNA and 50 mM phosphate buffer (pH 7.5)) is used.

Another specific example of the homologous DNA can include DNA encoding a protein having a base sequence including substitution, deletion, insertion, addition or inversion in one or a plurality of bases when the base sequence of SEQ ID NO.: 19 is a reference base sequence, and having an FAD-GDH activity. The substitution, deletion, or the like, of the base may occur in a plurality of sites. The "plurality" herein denotes, for example, 2 to 40 bases, preferably 2 to 20 bases, and more preferably 2 to 10 bases, although it depends upon the positions or kinds of the amino acid residue in the three-dimensional structure of the protein encoded by the DNA. The above-mentioned homologous DNA can be obtained by modifying DNA having the base sequence of SEQ ID NO.: 19 so that a certain site may include substitution, deletion, insertion, addition and/or inversion of base by using a site specific mutation method by, for example, a treatment with a restriction enzyme; treatment with exonuclease, DNA ligase, etc; introduction of mutation by a site-directed mutagenesis (Molecular Cloning, Third Edition, Chapter 13, Cold Spring Harbor Laboratory Press, New York); random mutagenesis (Molecular Cloning, Third Edition, Chapter 13, Cold Spring Harbor Laboratory Press, New York), and the like. Furthermore, homologous DNA can be obtained by other method such as irradiation with ultraviolet ray.

A further example of the homologous DNA can include DNA having difference in base as mentioned above due to polymorphism represented by SNP (single base polymorphism).

The gene of the present invention can be prepared in an isolated state by standard genetic engineering technique, molecular biological technique, biochemical technique, and the like, with reference to sequence information disclosed in this specification or attached sequence list. Specifically, the gene of the present invention can be prepared by appropriately using an oligonucleotide probe and a primer capable of specifically hybridizing the gene of the present invention from appropriate genome DNA library or cDNA library of filamentous fungi and yeasts, or cell body extract of filamentous fungi and yeasts. In general, an oligonucleotide probe and primer can be easily synthesized by using, for example, a commercially available automated DNA synthesizer, and the like. As to a production method of libraries used for preparing the gene of the present invention, see, for example, Molecular Cloning, Third Edition, Cold Spring Harbor Laboratory Press, New York.

For example, a gene having a base sequence set forth in SEQ ID NO: 19 can be isolated by using a hybridization method using an entire or a part of the complimentary sequence as a probe. Furthermore, by using a nucleic acid amplification reaction (for example, PCR) using a synthesized oligonucleotide primer designed to specifically hybridize to a part of the base sequence, amplification and isolation can be carried out.

(Vector)

Another aspect of the present invention relates to a vector containing the gene of the present invention. The term "vector," as used in this specification, is intended to refer to a nucleic acid molecule capable of transporting another nucleic acid which is inserted into the vector to the inside of the target such as cells. The type and form of vector is not particularly limited and therefor examples of the vector may include a plasmid vector, a cosmid vector, a phage vector, a viral vector (e.g., an adenovirus vector, an adeno-associated virus vector, a retrovirus vector, a herpes virus vector).

In accordance with the purpose of use (cloning, protein expression), and by considering the kinds of host cells, an appropriate vector is selected. Specific examples of a vector include a vector using Escherichia coli as a host (M13 phage or the modified body thereof, λ phage or the modified body thereof, pBR322 or the modified body thereof (pB325, pAT153, pUC8, etc.) and the like), a vector using yeast as a host (pYepSecl, pMFa, pYES2, etc.), a vector using insect cells as a host (pAc, pVL, etc.), a vector using mammalian cells as a host (pCDM8, pMT2PC, etc.).

The vector of the present invention is preferably an expression vector. The term "expression vector" is a vector capable of introducing the nucleic acid inserted therein into the target cells (host cells) and expressing in the cells. The expression vector usually includes a promoter sequence necessary for expression of the inserted nucleic acid and an enhancer sequence for promoting the expression, and the like. An expression vector including a selection marker can be used. When such an expression vector is used, by using the selection marker, the presence or absence of the introduction of an expression vector (and the degree thereof) can be confirmed.

Insertion of the gene of the present invention into a vector, insertion of the selection marker gene (if necessary), and insertion of a promoter (if necessary), and the like, can be carried out by a standard recombination DNA technology (see, for example, Molecular Cloning, Third Edition, 1.84, Cold Spring Harbor Laboratory Press, New York, a well-known method using restriction enzyme and DNA ligase).

(Transformant)

The present invention further relates to a transformant into which the gene of the present invention is introduced. In the transformant of the present invention, the gene of the present invention exists as a foreign molecule. The transformant of the present invention can be preferably obtained by transfection or transformation using the vector of the present invention mentioned above. The transfection and transformation can be carried out by, for example, a calcium phosphate coprecipitation method, electroporation (Potter, H. et al., Proc. Natl. Acad. Sci. U.S.A. 81, 7161-7165(1984)), lipofection (Felgner, P.L. et al., Proc. Natl. Acad. Sci. U.S.A. 84,7413-7417 (1984)), microinjection (Graessmann, M. & Graessmann, A., Proc. Natl. Acad. Sci. U.S.A. 73,366-370 (1976)), a method by Hanahan (Hanahan, D., J. Mol. Biol. 166, 557-580(1983)), a lithium acetate method (Schiestl, R.H. et al., Curr. Genet. 16, 339-346(1989)), a protoplast-polyethylene glycol method (Yelton, M.M. et al., Proc. Natl. Acad. Sci. 81, 1470-1474(1984)), and the like.

Examples of the host cell may include bacterial cell such as Escherichia coil, yeast cell (for example, Saccharomyces cerevisiae, Schizosaccharomyces pombe, Pichia pastoris), filamentous fungi cell (for example, Aspergillus oryzae, Aspergillus niger), and the like.

(Production method of FAD-GDH)

A further aspect of the present invention provides a manufacturing method of FAD-GDH. In one exemplary embodiment of the present invention, a manufacturing method includes a step of culturing microorganism having an ability to produce the enzyme (FAD-GDH) (step (1)); and a step of recovering flavin adenine dinucleotide-binding glucose dehydrogenase from a culture solution and/or fungus body after culturing (step (2)).

As the microorganism to be used in the step (1), for example, the above-mentioned Aspergillus oryzae BB-56, and the like, can be used. Culture methods and culture conditions are not particularly limited as long as the intended enzyme can be produced. That is to say, methods and culture conditions suitable for culturing microorganism to be used can be appropriately set in the conditions in which the enzyme is produced. Hereinafter, as the culture conditions, medium, culture temperature and culturing time will be described as an example.

As the medium, any medium can be used as long as microorganisms to be used can grow. For example, medium implemented with a carbon source such as glucose, sucrose, gentiobiose, soluble starch, glycerin, dextrin, molasses, and organic acid; and furthermore, a nitrogen source such as ammonium sulfate, ammonium carbonate, ammonium phosphate, ammonium acetate, or peptone, yeast extract, corn steep liquor, casein hydrolysate, bran, and meat extract; and furthermore, an inorganic salt such as potassium salt, magnesium salt, sodium salt, phosphate, manganese salt, iron salt, and zinc salt, and the like, can be used. In order to promote the growth of microorganisms to be used, vitamin, amino acid, and the like, may be added to medium. The medium is cultured under the aerobic conditions in which pH of the medium is adjusted to, for example, about 3 to 8 and preferably about 5 to 7; and the culture temperature is generally about 10 to 50°C and preferably about 25 to 35°C, for 1 to 15 days, preferably 3 to 7 days. An example of the culture method may include a shake culture method, and an aerobic submerged culture method by using a jar fermenter.

After culturing in the above-mentioned conditions, FAD-GDH is recovered from the culture solution or fungus body (step (2)). When FAD-GDH is recovered from the culture solution, the enzyme can be obtained by separation and purification by removing insoluble matters by, for example, filtration of culture supernatant, centrifugation, and the like, followed by carrying out an appropriate combination of concentration by ultrafilter membrane, salting out by ammonium sulfate precipitation, dialysis, various types of chromatography, and the like.

On the other hand, when FAD-GDH is recovered from fungus body, the enzyme can be obtained by pulverizing the fungus body by pressuring treatment, ultrasonic treatment, and the like, followed by separation and purification thereof similar to the above. Note here that after fungus body may be recovered from a culture solution by filtration, centrifugation, and the like, followed by carrying out the above-mentioned series of processes (pulverizing, separation, and purification of fungus body).

Note here that in each purification process, in principle, fraction is carried out using the FAD-GDH activity as an index and then the next step is carried out except in cases where an appropriate condition can be set by preliminary test, and the like.

In the other exemplary embodiment of the present invention, FAD-GDH is manufactured by using the above-mentioned transformant. In the manufacturing method in this exemplary embodiment, the above-mentioned transformant is cultured in the conditions in which protein encoded by a gene introduced therein is produced (step (i)). The culture conditions of transformant are well known as to various vector-host systems, and a person skilled in the art can easily set an appropriate culture condition. Following the culture step, the produced protein (that is, FAD-GDH) is recovered (step (ii)). The recovery and the subsequent purification may be carried out similar to the above-mentioned exemplary embodiment.

The purification degree of the enzyme is not particularly limited, but the enzyme can be purified so that the enzyme has a specific activity of, for example, 50 to 160 (U/mg), preferably, 100 to 160 (U/mg). Furthermore, the final form may be a liquid state or a solid state (including a powdery state).

(Application of FAD-GDH)

A further aspect of the present invention relates to an application of the enzyme. Firstly, this aspect provides a glucose measurement method using the enzyme. With the glucose measurement method of the present invention, the amount of glucose in a sample is measured by using oxidation reduction reaction by the enzyme. The present invention can be used for measurement of blood glucose level, glucose level in foods (seasoning agent, beverage, and the like). Furthermore, the present invention may be used for examining the fermentation degree in the manufacturing process of fermented food (for example, vinegar) or fermented beverage (for example, beer, liquor). In the enzyme, since FAD as coenzyme is always bound to GDH, a coenzyme is not required to be added during measurement, a simple measurement system can be constructed.

Furthermore, the present invention provides a reagent for measuring glucose, which contains the enzyme. The reagent is used in the glucose measurement method of the present invention.

The present invention further provides a kit (kit for measuring glucose) for carrying out the glucose measurement method of the present invention. The kit of the present invention includes a reagent for reaction, a buffer solution, a glucose reference solution as arbitrary components in addition to a reagent for measuring glucose. Furthermore, the kit for measuring glucose of the present invention generally includes instruction for use.

[EXAMPLES]

1. Screening of fungus producing FAD-GDH

(1-1) Culture of Aspergillus oryzae

Seven strains of Aspergillus oryzae (BB-56, IAM2603, IAM2628, IAM2683, IAM2736, IAM2706, and NBRC30113) were cultured by the following method.

(1-1-1) Preliminary culture

Fifty ml of culture medium having a composition including 0.2% (w/v) yeast extract (Becton, Dickinson Company), 1.0% (w/v) soy peptone (DMV), 2.0% (w/v) glucose (Wako Pure Chemical Industries, Ltd.), 0.1% (w/v) $KH_2PO_4$ (Wako Pure Chemical Industries, Ltd.), and 0.05% (w/v) (pH5.7) $MgSO_4 \cdot 7H_2O$ (SIGMA-ALDRICH Japan K.K.) was dispensed in a 300 mL Erlenmeyer flask, which was sterilized at 121°C at 0.12 MPa for 20 minutes, and cooled. After cooling, each strain of Aspergillus oryzae was inoculated in a slant (5 x 5 min) and preliminarily cultured at 30°C at 200 rpm for three days.

(1-1-2) Culture

Fifty ml of culture medium having a composition including 15.0% (w/v) glucose, 3.0% (w/v) Meast PIG (Asahi Food & Healthcare Co., Ltd.), 6.0% (w/v) soy peptone, 0.3% (w/v) $KH_2PO_4$, 0.2% (w/v) $K_2HPO_4$ (Wako Pure Chemical Industries, Ltd.), and 4 mM Hydroquinone (Wako Pure Chemical Industries, Ltd.) (pH 6.0) was dispensed in a 300 mL Erlenmeyer flask, which was sterilized at 121 °C at 0.12 MPa for 20 minutes, and cooled. After cooling, 1 mL of culture solution of the above mentioned preliminary culture was inoculated and cultured at 30°C at 200rpm for four days. After culturing, the culture solution was filtrated by using filter paper No. 2 (Advantech Co., Ltd.) and then the presence of the FAD-GDH activity of the filtrate of each strain was confirmed.

(1-2) Detection of FAD-GDH activity

FAD-GDH catalyzes a reaction of oxidizing a hydroxyl group of glucose in the presence of an electron acceptor to yield glucono-δ-lactone. The detection of the FAD-GDH activity was carried out by the following reaction system.

[Formula 1]

(1) D-glucose + PMS $\rightarrow$ D- glucono-δ-lactone + reduced PMS

(2) 2 reduced PMS + NTB $\rightarrow$ 2PMS + Diformazan

In the formula, PMS denotes Phenazine methosulfate, and NTB denotes Nitrotetrazorium blue.
In the reaction (1), the reduced PMS is produced according to the oxidation of glucose. Furthermore, Diformazan produced by the reduction of NTB by the reduced PMS in the reaction (2), is measured in the wavelength of 570 nm.
The enzyme activity (unit) is calculated from the following calculation formula.

[Formula 2]

$$EnzymeActivity(U/mL) = \frac{\Delta OD/\min(\Delta OD_{test} - \Delta OD_{blank}) \times Vt \times df}{20.1 \times 1.0 \times Vs}$$

In the formula, Vt denotes a total amount of solution, Vs denotes an amount of sample, 20.1 denotes absorption coefficient per 0.5 μmole of diformazan ($cm^2$/0.5μmole), 1.0 denotes optical path length (cm), and df denotes dilution ratio, respectively.
2.55 mL of 50 mM PIPES-NaOH buffer solution (pH 6.5) containing 0.22% (w/v) Triton X-100, 0.09 mL of 1 M D-glucose solution, 0.2 mL of 3 mM PMS solution, and 0.1 mL of 6.6 mM NTB solution were mixed with each other, kept at 37°C for 5 minutes, and thereafter, 0.1 mL of the culture filtrate was added to the mixture so as to start the reaction. Along with the progress of the enzyme reaction, Diformazan having the absorption in 570nm is produced. By measuring the increase per minute of the absorbance in 570 nm, the FAD-GDH activity was measured.
As a result, the FAD-GDH activity was detected in the culture filtrate derived from Aspergillus oryzae BB-56, IAM2603, IAM2628, IAM2683, IAM2736, IAM2706, and NBRC30113 (see Table 1). In particular, the productivities of Aspergillus oryzae BB-56 and NBRC30113 were excellent.
[Table 1]

Table 1
Screening of fungus producing FAD-GDH

| Name of fungus | Activity (u/mL) |
|---|---|
| Aspergillus oryzae BB-56 | 1.97 |
| Aspergillus oryzae IAM2063 | 0.11 |
| Aspergillus oryzae IAM2628 | 0.87 |
| Aspergillus oryzae IAM2683 | 0.41 |
| Aspergillus oryzae IAM2735 | 0.20 |
| Aspergillus oryzae IAM2706 | 0.75 |
| Aspergillus oryzae NBRC30113 | 2.12 |

2. Production of FAD-GDH and Purification of enzyme

(2-1) Culture of strain

Aspergillus oryzae BB-56 was cultured so as to produce FAD-GDH. The medium for preliminary culture having a composition including 0.2% (w/v) yeast extract, 0.5% (w/v) soy peptone, 2.0% (w/v) glucose, 0.1 % (w/v) $KH_2PO_4$, and 0.05% (w/v) $MgSO_4 \cdot 7H_2O$ (pH5.7) was prepared. This liquid medium (50 mL) was dispensed in a 300 mL Erlenmeyer flask, which was sterilized at 121 °C at 0.12 MPa for 20 minutes. Then, each strain of Aspergillus oryzae BB-56 was inoculated and cultured at 30°C at 200 rpm for three days.
FAD-GDH was produced in the culture medium having the following composition. The composition includes 10.0% (w/v) glucose, 2.0% (w/v) Meast PIG, 4.0% (w/v) soy peptone, 0.3% (w/v) $KH_2PO_4$, 0.2% (w/v) $K_2HPO_4$ and 4 mM Hydroquinone (pH 6.0). This culture medium (20 L) was placed in a 30 L jar fermenter, which was sterilized at 121 °C at 0.12 MPa at 200 rpm for 20 minutes, and cooled to 30°C. The above-mentioned culture solution for preliminary culture (200 mL) was inoculated in the culture medium and cultured at 30°C at 350 rpm at 0.05 MPa and in the ventilation volume of 0.75 vvm for four days. Thus, the FAD-GDH was produced.

(2-2) Purification of FAD-GDH

The thus obtained culture solution (15.0 L) in 30Ljar fermenter was subjected to filtration of diatomaceous earth using silica #600S (Chuo Silica Co., Ltd.) so as to remove fungus bodies and other solid components in the culture solution. The purified solution (16.4 L) obtained by the above-mentioned operation was desalinated and concentrated to 3.0 L by using an ultrafiltration membrane (ACP-3013, 13,000 cut, Asahi Kasei Corporation, the same is true hereinafter). The concentrated solution was subjected to salting out with 90% saturated ammonium sulfate. The centrifuged supernatant was desalinated (10 mmol/L McIlvaine buffer, pH 5.5) and concentrated by using an ultrafiltration membrane so as to adjust to pH 5.5 and conductivity of 1.10 mS/cm. The desalinated and concentrated solution was applied to CM-Sepharose Fast Flow (column volume of 1,000 mL, Amersham Biosciences) that was equilibrated with 10 mmol/L McIlvaine buffer (pH 5.5) so that a column was allowed to adsorb FAD-GDH. The column was washed with 10 mmol/L McIlvaine buffer (pH 5.5), and then FAD-GDH was eluted with 10 mmol/L McIlvaine buffer (pH 5.5) containing 0.1 mol/L NaCl. Then, FAD-GDH active fractions were recovered. The recovered active fractions were concentrated by using ultrafiltration membrane. The concentrated solution was applied to Octyl-Sepharose (column volume: 200 mL) equilibrated with 10 mmol/L $K_2HPO_4$-NaOH buffer (pH 6.5) containing 2.5 mol/L ammonium sulfate so that a column was allowed to adsorb FAD-GDH. The column was washed with 10 mmol/L $K_2HPO_4$-NaO buffer (pH 6.5) containing 2.5 mol/L ammonium sulfate and then the concentration of ammonium sulfate in the buffer was changed stepwise to 2.0, 1.5, 1.0, and 0.5 mol/L. Thereby, FAD-GDH was eluted. The active fractions of FAD-GDH were recovered and then were desalinated (20 mmol/L $K_2HPO_4$-NaOH buffer, pH 6.5) with ultrafiltration membrane and concentrated. The desalinated and concentrated solution was applied to DEAE-Sepharose CL-6B (column volume of 50 mL, Amersham Biosciences) that was equilibrated with 20 mmol/L $KH_2PO_4$-NaOH buffer (pH 6.5) so as to elute FAD-GDH with 20 mmol/L $KH_2PO_4$-NaOH buffer (pH 6.5). This fraction was desalinated (10 mmol/L $KH_2PO_4$-NaOH, pH 6.5) with ultrafiltration membrane and concentrated. Thus, purified enzyme preparation of FAD-GDH was obtained. As shown in Table 2, the yield of the purified enzyme was 14% with respect to the diatomaceous earth filtrate and the specific activity was increased to about 41,000 times.

[Table 2]

Table 2

| Purification of FAD-GDH | | | | | | |
|---|---|---|---|---|---|---|
| | amount of solution(mL) | FAD-GDH activity | | | specific activity (u/mg protein) | purification ratio |
| | | U/mL | total activity (u) | yield(%) | | |
| diatomaceous earth filtrate | 18400 | 1.31 | 21500 | 100 | 0.003 | 1 |
| UF concentrated Solution | 3000 | 7.49 | 22500 | 105 | 0.030 | 9 |
| 90% saturated ammonium sulfate salting-cut supernatant | 5000 | 2.95 | 14800 | 68.8 | 0.026 | 7 |
| UF desalinated concentrated solution | 1000 | 23.8 | 23800 | 111 | 1.43 | 417 |
| CM-Sepharose elute | 2000 | 4.39 | 8780 | 40.8 | 3.99 | 1163 |
| UF desalinated concentrated solution | 320 | 28.6 | 9150 | 42.6 | 4.39 | 1280 |
| Octyl-Sepharose elute | 400 | 11.3 | 4520 | 21.0 | 31.9 | 9300 |
| UF desalinated concentrated solution | 35 | 104 | 3840 | 16.9 | 31.1 | 9067 |
| DEAE-Sepharose elute | 135 | 25.5 | 3440 | 16.0 | 135 | 39359 |

(continued)

| Purification of FAD-GDH | | | | | | |
|---|---|---|---|---|---|---|
| | amount of solution(mL) | FAD-GDH activity | | | specific activity (u/mg protein) | purification ratio |
| | | U/mL | total activity (u) | yield(%) | | |
| UF desalinated concentrated solution | 5 | 620 | 3100 | 14.4 | 142 | 41399 |

3. Measurement of molecular weight

(3-1) Measurement of molecular weight of FAD-GDH by sodium dodecyl sulfate-polyacrylamide - gel electrophoresis (SDS-PAGE)

The molecular weight of purified FAD-GDH derived from Aspergillus oryzae BB-56 was measured by SDS-PAGE. The measurement was carried out by Phast System (GE Healthcare Biosciences) in which homogeneous 12.5 (GE Healthcare Bio-Sciences) was used for gel and Protein Molecular Markers 14.4-97 kDa (GE Healthcare Bio-Sciences) was used for the molecular weight marker. A band of protein on the gel was stained with PhastGel Blue R (Coomassie R350 staining) so as to detect protein. As a result, a single band was detected around molecular weight 100 kDa (see, Fig. 1).

(3-2) Measurement of molecular weight of FAD-GDH by gel filtration chromatography

The molecular weight of purified FAD-GDH derived from Aspergillus oryzae BB-56 was measured by gel filtration chromatography by HPLC (LC-10 AD VP, Shimadzu Corporation). 25 μl of purified FAD-GDH solution was applied to TSK-gel G3000SW (7.5 mm I.D. x 30cm, Tosoh Corporation) equilibrated with 50 mM phosphate buffer (pH 6.9) containing 0.3M NaCl, eluted with the buffer at a column temperature of 25°C and flow rate of 0.7 mL/min so as to measure the absorbance in 280 nm. As a result of formation of a calibration curve by the molecular weight marker (MW-Marker proteins, Oriental Yeast Co., Ltd), purified FAD-GDH is shown to be a protein having the molecular weight of about 400,000 (Fig. 2).

The molecular weights of FAD-GDH produced by strains of Aspergillus oryzae BB-56, IAM2603, IAM2628, IAM2683,IAM2736, IAM2706, and NBRC30113 were compared with each other. 3 mL each of culture filtrate of Aspergillus oryzae BB-56, 1AM2603, IAM2628, IAM2683, IAM2736, IAM2706, and NBRC30113 was applied to Sephadex G-100 (diameter: 2.2 cm x height 105 cm, column volume: 360 mL, GE Healthcare Bio-Sciences) equilibrated with 0.1M $KH_2PO_4$-NaOH buffer (pH 6.5) so as to allow the column to charge FAD-GDH. The charged FAD-GDH was eluted with the above-mentioned buffer. Then, the eluted fractions were recovered and the FAD-GDH activity of each fraction was measured by the above-mentioned method 1. As a result of formation of a calibration curve by the molecular weight marker, in every culture filtrate, the FAD-GDH activity was detected at the positions having a molecular weight of about 400,000 (Fig. 3). Thus, FAD-GDH derived from Aspergillus oryzae BB-56, IAM2603, IAM2628, IAM2683, IAM2736, IAM2706, and NBRC30113 are shown to be proteins having the molecular weight of 400,000.

4. Absorption Spectrum

The purified FAD-GDH derived from A. oryzae BB-56 was diluted with 10 mM phosphate buffer (pH 6.5) and the absorption spectrum in 400-800 nm was scanned by the use of an absorbance meter (Shimadzu Corporation). As a result, the maximum absorption was observed around 460 nm peculiar to the flavin enzyme, so that the enzyme was confirmed to be a flavin-binding protein (Fig. 4).

5. Optimum pH, temperature, and pH and temperature for stability of FAD-GDH

(5-1) Investigation of optimum pH

2.55 mL of 100 mM Mcllvaine buffer (adjusted to pH4.5, pH 5.0, pH 6.0, pH 7.0, or pH 8.0) containing 0.22% (w/v) Triton X-100, 0.1 mL of 1M D-glucose solution, 0.2 mL of 3mM PMS solution and 0.1 mL of 6.6 mM NTB solution were mixed with each other and kept at 37°C for 5 minutes. Then, 0.1 mL of the purified FAD-GDH solution derived from Aspergillus oryzae BB-56 was added to the mixture so as to start reaction. Diformazan produced by the enzyme reaction was measured in the absorbance in 570nm and the amount Diformazan produced per minute was measured. Thus, the enzymatic activity was measured. The optimum pH of purified FAD-GDH derived from Aspergillus oryzae BB-56 was 7.0 (Fig. 5).

(5-2) Investigation of Optimum Temperature

2.55mL of 50 mM PIPES-NaOH buffer (pH 6.5) containing 0.22% (w/v) Triton X-100, 0.1 mL of 1M D-glucose solution, 0.2 mL of 3mM PMS solution and 0.1 mL of 6.6 mM NTB solution were mixed with each other and kept at 30, 37, 45, 55, 60 or 65°C for 5 minutes. Then, 0.1 mL of the purified FAD-GDH solution derived from Aspergillus oryzae BB-56 was added to the mixture so as to start the reaction at 30, 37, 45, 50, 55, 60 or 65°C. Diformazan produced by the enzyme reaction was measured in the absorbance in 570 nm and the amount Diformazan produced per minute was measured. Thus, the enzymatic activity was measured. The optimum temperature of the purified FAD-GDH derived from Aspergillus oryzae BB-56 was about 60°C (Fig. 6).

(5-3) Investigation of pH for stability

A purified FAD-GDH solution was diluted (50 U/mL) with each buffer, i.e., 0.1M acetate buffer (pH3, pH4), 0.1M McIlvaine buffer (pH 4.5, pH 5.0, pH 6.0, pH 7.0, pH 8.0), or 0.1M $Na_2CO_3$-$NaHCO_3$ buffer (pH 9.5) and treated at 37°C for 30 minutes at each pH. Each treatment solution was appropriately diluted with 0.2M $KH_2PO_4$-NaOH buffer (pH 6.5) and the FAD-GDH activity was measured by the above-mentioned method 1. As a control, the activity of purified FAD-GDH, which was diluted with 0.1M $KH_2PO_4$-NaOH buffer (pH 6.5) and stored in ice, was also measured. As a result, FAD-GDH maintains 80% or more of the activity at least in the range from pH 3.0 to pH 7.0, showing that FAD-GDH is stable in the range from pH 3.0 to pH 7.0 (Fig. 7).

(5-4) Investigation of Temperature for Stability

Purified FAD-GDH was diluted to I U/mL with 50 mM PIPES-NaOH buffer (pH 6.5) containing 1 mM calcium chloride, 0.1% (w/v) Triton X-100, and 0.1% (w/v) bovine serum albumin and treated at each designated temperature (5, 30, 40, 50, or 60°C) for 20 minutes. Each treated solution was cooled in ice and then it was diluted with 50mM PIPES-NaOH buffer (pH 6.5) containing 1 mM calcium chloride, 0.1% (w/v) Triton X-100, 0.1% (w/v) bovine serum albumin, and the FAD-GDH activity was measured in the above-mentioned method 1. As a control, FAD-GDH of the not thermally treated enzyme was measured. As a result, FAD-GDH maintained substantially 100% of activity at 40°C or less, showing that FAD-GDH is stable at 40°C or less (Fig. 8).

6. Measurement of isoelectric point

3 mL of the purified FAD-GDH solution derived from Aspergillus oryzae BB-56 was placed in a dialysis membrane 36 (Wako Pure Chemical Industries, Ltd.), which was immersed in purified water and dialyzed at 4°C over night. This dialysis sample was subjected to glycerol density gradient electrophoresis. A carrier ampholyte having a column volume of 110 mL (Pharmalyte™ 3-10 for IEF (Amersham Biosciences AB), average final concentration of 1%) was used to allow an electric current to pass at 5°C at a constant voltage of 400V for 48 hours. After passing current, samples were recovered at the flow rate of about 2 mL/min (one fraction was made to be 1.5 mL). When pH and FAD-GDH activity of each fraction were measured, pI of FAD-GDH derived from Aspergillus oryzae BB-56 was 6.4 (Fig. 9).

7. Substrate Specificity

(7-1) Investigation of Substrate Specificity 1 (Substrate Specificity of Purified FAD-GDH)

2.55mL of 50 mM PIPES-NaOH buffer (pH 6.5) containing 0.22% (w/v) Triton X-100, 0.09 mL of 1M substrate (D-glucose, maltose, galactose, xylose, maltotriose, maltohexaose, or maltopentaose) solution, 0.2 mL of 3 mM PMS solution, and 0.1 mL of 6.6 mM NTB solution were mixed with each other and kept at 37°C for 5 minutes. Thereafter, 0.1 mL of purified FAD-GDH solution was added to the mixture so as to start the reaction. Diformazan produced by the enzyme reaction was measured in the absorbance in 570nm and the amount Diformazan produced per minute was measured. Thus, the enzymatic activity was measured. The relative activity with respect to each substrate was calculated when the reaction rate with respect to D-glucose was assumed to be 100% (Table 3).

[Table 3]

Table 3

Relative activity to each substrate of purified FAD-GDH

| substrate | relative activity(%) |
|---|---|
| glucose | 100 |
| maltose | 0.582 |
| galactose | 0.349 |
| xylose | 22.7 |
| maltotriose | 0.239 |
| maltohexaose | 0.479 |
| maltopentaose | 4.55 |

(7-2) Investigation of Substrate Specificity 2 (Comparison of Substrate Specificity of FAD-GDH produced by each strain)

(7-2-1) Preparation of enzyme sample

10 mL of each culture filtrate derived from Aspergillus oryzae BB-56, IAM2603, IAM2628, IAM2683, IAM2736, IAM2706, or NBRC30113 was placed in a dialysis membrane 36 and dialyzed in 10 mM $KH_2PO_4$-NaOH at 4°C over night.

(7-2-2) Measurement of Relative Activity

2.5 mL of 20 mM MOPS buffer (pH 7.0) containing 2 mM calcium chloride, and 0.3 mL of 0.4M substrate solution (D-glucose, 2-deoxyglucose, xylose, fructose, mannose, or maltose) were mixed with each other and kept at 25°C for 5 minutes. Then, 0.05 mL of 20mM PMS solution, 0.05 mL of 4 mM DCIP (2,6-dichloroindophenol) solution, and 0.1 mL of each dialysis sample were added to the mixture so as to start an enzyme reaction. By measuring the decrease of DCIP by the reduction reaction in the absorbance at 600 nm, the FAD-GDH activity was measured. The activity with respect to each substrate was calculated when the activity with respect to D-glucose as a substrate was assumed to be 100% (Table 4). From the results, the substrate specificities of FAD-GDH derived from Aspergillus oryzae BB-56, IAM2603, IAM2628, IAM2683, LAM2736, IAM2706, or NBRC30113 are the same as each other.
[Table 4]

Table 4

Comparison of relative activity to each substrate

| substrate | relative activity(%) | | | | | | |
|---|---|---|---|---|---|---|---|
| | BB-56 | IAM2603 | IAM2628 | IAM2683 | IAM2736 | IAM2706 | NBRC301 13 |
| glucose | 100 | 100 | 100 | 100 | 100 | 100 | 100 |
| 2-deoxyglucose | 35.1 | 29.0 | 34.2 | 32.7 | 31.3 | 33.9 | 35.3 |
| xylose | 17.6 | 16.1 | 16.4 | 17.3 | 14.6 | 16.1 | 17.6 |
| fructose | 0 | 0 | 1.40 | 0 | 0 | 0 | 0 |
| mannose | 1.40 | 0 | 2.70 | 1.90 | 2.10 | 0 | 1.20 |
| maltose | 0 | 0 | 0 | 0 | 0 | 0 | 0 |

8. Measurement of Michaelis-Menten, Km constant

2.55 mL of 50mM PIPES-NaOH buffer (pH 6.5) containing 0.22% (w/v) Triton X-100, 0.1 mL of 6.1-610 mM D-glucose solution, 0.2 mL of 3 mM PMS solution, and 0.1 mL of 6.6 mM NTB solution were mixed with each other and kept at 37°C for 5 minutes. Then, 0.1 mL of purified FAD-GDH solution was added to the measuring the amount of Diformazan per minute. When Km value was calculated by Lineweaver-burk plot (Fig. 10), the Km value with respect to D-glucose of purified FAD-GDH was 8.2 mM.

9. Sugar Content

The content of sugar per enzyme protein was measured by a phenol sulfuric acid method. 1.0 mL of purified FAD-GDH solution, 1.0 mL of 5% (w/v) phenol solution, 5.0 mL of 98% sulfuric acid were mixed with each other and stood still at room temperature for 20 minutes. After the mixture was cooled by flowing water, absorbance in 490 nm was measured. Calibration curve was formed by 0-0.03 mg/mL D-glucose standard solution (Fig. 11) and the glucose content per 1 mg of enzyme was calculated. As a result, the sugar content of FAD-GDH was 0.43 mg (43%) per 1 mg of enzyme.

10. Comparison with respect to glucose oxidase (GO)

(10-1) Measurement of FAD-GDH activity

2.55mL of 50 mM PIPES-NaOH buffer (pH 6.5) containing 0.22% (w/v) Triton X-100, 0.1 mL of 1M D-glucose solution, 0.2 mL of 3mM PMS solution, and 0.1 mL of 6.6mM NTB solution were mixed with each other and kept at 37°C for 5 minutes. 0.1 mL of appropriately diluted enzyme solution (purified FAD-GDH derived from Aspergillus oryzae BB-56, or GO (AMANO ENZYME INC)) was added to the mixture so as to start reaction at 37°C. Diformazan produced by the enzyme reaction was measured in the absorbance in 570nm and the amount Diformazan produced per minute was measured. Thus, the enzymatic activity was measured.

(10-2) Measurement of GO activity

The glucose oxidase activity was measured in the following reaction principle. [Formula 3]

Reaction 1: D-glucose + $O_2$ $\rightarrow$ D- glucono-$\delta$-lactone + $H_2O_2$

Reaction 2: $H_2O_2$ + 4-aminoantipyrine + phenol $\rightarrow$ quinonimine dye + $4H_2O$

$H_2O_2$ produced with GO in the reaction 1 is used in a production reaction of quinonimine dye with peroxidase in the reaction 2. By measuring the quinonimine dye in the absorbance in 500 nm, the GO activity was measured. The amount of enzyme oxidizing 1 $\mu$mole of D-glucose for one minute in the reaction condition at 37°C is defined as one unit of the GO activity. The actual measurement method is shown below.

2.0 mL of phenol solution (0.1M phosphate buffer (pH 7.0) containing shown below.

2.0 mL of phenol solution (0.1M phosphate buffer (pH 7.0) containing 0.152% phenol and 0.152% Triton X-100), 0.5 mL of 0.555 mol/L D-glucose solution, 0.5 mL of 25 U/mL peroxidase (AMANO ENZYME INC) solution, and 0.1 mL of 4 mg/mL 4-aminoantipyrine solution were mixed with each other and kept at 37°C for 5 minutes. Then, 0.1 mL of appropriately diluted enzyme solution (purified FAD-GDH derived from Aspergillus oryzae BB-56, or GO) was added to the mixture so as to start reaction at 37°C. Two minutes and 5 minutes after the reaction was started, the absorbance in 500 nm was measured. Furthermore, as enzyme blank, 0.1 M phosphate buffer (pH 7.0) was added instead of an enzyme solution, similarly the absorbance was measured. The GO activity was calculated from the following calculation formula.

[Formula 4]

$$\text{GO Activity (U/mg)} = \frac{(A5 - A2) - (Ab5 - Ab2)}{3} \times \frac{1}{12.88 \times 1/2} \times 3.2 \times \frac{Dm}{0.1}$$

In the formula, "3" denotes a reaction time, "12.88" denotes molecular absorption constant (mM) of the quinonimine dye, "1/2" denotes a coefficient based on the fact that 1 mole of quinonimine dye is produced from 2 mole of $H_2O_2$, "3.2" denotes a final liquid amount of the reaction solution, "0.1" is sample amount (mL), and "Dm" is dilution ratio, respectively.

The results shows that the purified FAD-GDH derived from Aspergillus oryzae BB-56 exclusively has the GDH activity and does not substantially have the GO activity. On the other hand, it was determined that GO mainly has the GO activity and it also has the GDH activity (Table 5). That is to say, it was shown that FAD-GDH derived from Aspergillus oryzae BB-56 is less susceptible to dissolved oxygen of the reaction system as compared with GO when D-glucose is measured by the use of a material of the electron transport system.

[Table 5]

Table 5

| Comparison between FAD-GDH and GO | | | |
|---|---|---|---|
| | | CDH activity | GO activity |
| Aspergillus oryzae | BB-56 FAD-GDH | 119U/mL | 0. 0025U/mL |
| GO | | 8. 86U/mL | 67. 8U/mL |

11. Measurement of Glucose level

Examples of the use of FAD-GDH derived from Aspergillus oryzae in accordance with the present invention are shown below. 2.55 mL of 50 mM PIPES-NaOH buffer solution (pH 6.5) containing 0.22% Triton X-100, 0.10 mL of D-glucose solution (0, 100, 200, 400, 600, 800, 1000 mg/dL), 0.20 mL of 3 mM PMS solution, and 0.10 mL of 6.6 mM NTB solution were mixed with each other and stood still at 37°C for 5 minutes. Then, 0.10 mL of FAD-GDH enzyme solution derived from Aspergillus oryzae BB-56 (0.3 U/mL) was added to the mixture so as to start the reaction. Diformazan produced by the enzyme reaction was measured in the absorbance in 570 nm. The relation between the increase per 3 minutes of the absorbance in 570 nm and the glucose level is shown in Fig. 12. It is shown that FAD-GDH derived from Aspergillus oryzae can accurately measure the glucose level in a sample when the glucose level is 800 mg/dL or less.

12. Inhibition test

The enzymatic activities were measured in the control group and the test group (group in which 1,10-phenanthroline was added) and both activities were compared with each other. Thereby, the inhibition effect of 1,10-phenanthroline on purified FAD-GDH was examined.

(Measurement of enzymatic activity in control group (see, Table 6))
[Table 6]

Table 6

| Measurement method of control group | |
|---|---|
| 0.1M $KH_2PO_4$-NaOH pH7. 0 | 1.0mL |
| 1M glucose solution(-1) | 1.0mL |
| 3mM DCIP solution | 0.1mL |
| 3mM 1-methoxy PMS solution | 0.2mL |
| purified water | 0.65mL |
| 37°C. 5min | preheating |
| sample | 0.05mL |
| 37°C. 600nm | (A1-A3) |
| *1) diluted buffer :0.1M $KH_2PO_4$-NaOH, pH7.0 | |

Firstly, 1.0 mL of 0.1 M potassium phosphate buffer (pH 7.0), 1.0 mL of 1 M D-glucose solution, 0.1 mL of 3mM DCIP (2,6-dichlorophenolindophenol), 0.2 mL of 3 mM 1-methoxy PMS (5-methylphenazinium methyl sulfate) solution, and 0.65 mL of purified water were placed in a quartz cell and kept at 37°C for 5 minutes. Subsequently, 0.05 mL of enzyme solution was added, the change of absorbance in 600 nm of DCIP (ΔABS/min) was measured, and the enzymatic activity was calculated by the following calculation formula.

**[Formula 5]**

$$\text{Enzyme Activity (unit/ml)} = \frac{-\Delta ABS}{16.3} \times \frac{3.0}{0.05} \times \text{Enzyme Dilution Ratio}$$

(Measurement of enzymatic activity in test group (see, Table 7))
[Table 7]

Table 7 Measurement method of test group (inhibitor-added group)

| | | | |
|---|---|---|---|
| 0.1M | $KH_2O_4$-NaOH pH7.0 | | 1.0mL |
| 1M | glucose solution | | 1.0mL |
| 3mM | DCIP solution | | 0.1mL |
| 3mM | 1-methoxy PMS solution | | 0.2mL |
| | purified water | | 0.35mL |
| each concentration 1.10-phenanthroline solution | | | 0.3mL |
| | | 37°C. 5min | preheating |
| | sample | | 0.05mL |
| | | 37°C. 600nm | (A1-A3) |

In the above-mentioned measurement method, the enzymatic activities when 1,10- phenanthroline was added so that the final concentration became 1 mM, 5 mM, 10 mM, 25 mM, and 50 mM were measured, respectively.

(Test result)

As shown in Table 8, the inhibition effect of 1,10-phenanthroline on the purified FAD-GDH was low and only about 2% inhibition effect in 1mM 1,10-phenanthroline was observed.
[Table 8]

Table 8

| Final concentration of 1,10-phenanthroline solution(mM) | Inhibition effect of PAD-GDH derived from Aspergillus oryzae(%) |
|---|---|
| 0 | 0 |
| 50 | - |
| 25 | - |
| 10 | 38 |
| 5 | 21 |
| 1 | 2 |

13. Identification of Aspergillus oryzae BB-56 strain

(1) Method

(1-1) Strain

Aspergillus oryzae BB-56

(1-2) Composition of medium

The following medium was prepared with reference to the report in Klich, M. A. (2002). Identification of Common Aspergillus species. Utrecht: Centraalbureau voor Schimmelcultures, 116 pp.
CYA medium: 1 g of $K_2HPO_4$, 10 mL of Czapek Concentrate, 5 g of Yeast extract (Difco), 30 g of Sucrose, 15g of agar (Wako), 1000 mL of purified water
CZ medium: 1 g of $K_2HPO_4$, 10 mL of Czapek Concentrate, 30 g of Sucrose, 17.5 g of agar (Wako), 1000 mL of purified water
CY20S medium: 1g of $K_2HPO_4$, 10 mL of Czapek Concentrate, 5 g of Yeast extract (Difco), 200 g of Sucrose, 15 g of agar (Wako), 1000 mL of purified water
MEA medium: 20 g of Malt extract (Difco), 20 g of Glucose, 1 g of Bacto-peptone (Difco), 15 g of Agar (Wako), 1000 mL of purified water
PDA medium: Potato dextrose agar (Eiken) was used.
Czapek Concentrate: 30 g of $NaNO_3$, 5 g of KCl, 5g of $MgSO_4 \cdot 7H_2O$, 0.1 g of $FeSO_4 \cdot 7H_2O$, 0.1 g of $ZnSO_4 \cdot 7H_2O$, 0.05 g of $CuSO_4 \cdot 5H_2O$, 100 mL of purified water

(1-3) Phenotype

The degree of growth was determined by measuring the diameter of the colony cultured at 25°C or 37°C for 7 days.

The growth state was observed with respect to CYA medium, CZ medium, CY20S medium, and MEA medium (25°C for 7 days).

The observation of the form was carried out in the culture of CYA medium at 25°C (cultured for 6 - 21 days).

The surface of conidium was observed by scanning electron microscopy after culturing using CYA medium at 25°C for 15 days followed by treatment by osmic acid vapor fixation.

The color of the colony was determined according to Standard Color Chart for horticulture plants in Japan (published by Japan Color Research Institute).

(2) Result

(2-1) Degree of Growth (diameter of colony)

The degrees of growth in various media (cultured for 7 days) are shown in the following Table.
[Table 9]

Table 9

| Growth degrees in various media (cultured for 7 days) | | |
|---|---|---|
| media | Aspergillus oryzae BB-56 | |
| | 25°C | 37°C |
| CYA | 59 - 62 mm | 3 - 5 mm |
| CZ | 33 - 38 mm | 2 - 3 mm |
| CY20S | 49 - 56 mm | 3 - 8 mm |
| MEA | 52 - 64 mm | 2 - 3 mm |
| PDA | 44 - 54 mm | 2 - 4 mm |

(2-2) Growth State

The growth states in various media (cultured at 25°C for 7 days) are shown in the following Table. Note here that color is determined according to Standard Color Chart for horticulture plants in Japan (published by Japan Color Research Institute) (numeric values in parentheses represent the color chart numbers).
[Table 10]

Table 10

| Growth states in various media (cultured at 25 °C for 7 days) | | | | | | | |
|---|---|---|---|---|---|---|---|
| Items to be observed | | | CYA | CZ | CY20S | MEA | PDA |
| surface of colony | shape of colony | | circular | circular | circular | circular | circular |
| | texture | | fluff-state | fluff-state | fluff-state | fluff-state | fluff-state |
| | surface unevenness | | radial wrinkle | weak irregular wrinkle | radial wrinkle | flat | flat |
| | formation of conidium | | rich | rich | moderate | slight | rich |
| | color | center portion | thick green yellow (2708) or dark green yellow (2712) | thick green yellow (2708) or dark green yellow (2712) | thick green yellow (2708) or dark green yellow (2712) | white | dark yellow green (3308) or moderate yellow green (3312) |
| | | middle portion | thick green yellow (2708) or dark green yellow (2712) | light green yellow (2703) | white | light green yellow (2703) | dark yellow green (3308) or moderate yellow green (3312) |
| | | peripheral portion | white | white | white | white | white |
| color of rear surface of colony | | | light yellow (2503) | light yellow (2503) | light yellow (2503) | light yellow (2503) | light yellow (2503) |
| water-soluble dye | | | None | None | None | None | None |

(2-3) Form

With reference to Raper, K. B. & Fennell, D. I. (1965). The genus Aspergillus. Williams & Wilkins Co., Baltimore, 686 pp., Kozakiewicz, Z. (1989). Aspergillus species on stored products. Mycological Papers. No.161: 1-188., Klich, M. A. (2002). Identification of Common Aspergillus species. Utrecht : Centraalbureau voor Schimmelcultures, 116 pp., Samson, R. A. Hoekstra, E. S. Frisvad, J. C. & Filtenborg, O. (2002). Introduction to food- and airborne fungi, 6th edn, pp.64-97. Utrecht : Centraalbureau voor Schimmelcultures, 389 pp., Aspergillus oryzae BB-56 has the following features. The color of the colony is thick green yellow or dark green yellow (Table 7); the degree of growth is 59 - 62 mm in the CYA medium (cultured at 25°C for 7 days) and 52 - 64 mm in the MEA medium (cultured at 25°C for 7 days) (Table 6); a conidial head was radial shape and loose column shape, a conidiophore is not constricted below the vesicle; single row and double rows of Aspergilla are coexisting; the vesicle has sub-spherical shape or flask shape with the diameter of 10 - 42 μm, and a conidium has sub-spherical or spherical shape with the size of 5.6 - 8.8 x 5.2 - 8.8 μm whose surface is smooth or slightly rough (Table 8), showing that the colony is Aspergillus oryzae.
[Table 11]

Table 11

| Form in CYA medium | | |
|---|---|---|
| Items to be observed | | Aspergillus oryzae BB-56 |
| conidiophore | shape | mainly from base mycelium, not constricted below the vesicle |
| | surface | smooth surface - slightly rough surface |
| | diameter | 5.6-16 μm |
| conidial head | | radial shape and loose column shape |

(continued)

| Form in CYA medium | | |
|---|---|---|
| Items to be observed | | Aspergillus oryzae BB-56 |
| Aspergilla | | single row and double rows of Aspergilla are coexisting, irregular Aspergilla is often observed |
| vesicle | shape | sub-spherical - flask shape, metula or phialide is adhered to the 1/3 - 3/4 of upper part |
| | diameter | 10.4-42 μm, reaches 59 μm but uncommonly |
| metula | length | 8.8-16 μm |
| | diameter | 5.6-8.0 μm (12 μm in irregular shape) |
| phialide | length | 8.4-16.8 μm |
| | diameter | 3.2-4.0 μm (8 μm in irregular shape) |
| conidium | shape | sub-spherical - spherical shape |
| | surface | slightly rough |
| | length | 5.6-8.8 μm |
| | diameter | 5.2-8.8 μm |
| ascospore | | Not formed |
| sclerotium | | Not formed |

14. Identification of FAD-GDH derived from Aspergillus oryzae BB-56

(1) Analysis of amino acid sequence

Aspergillus oryzae BB-56 was cultured, and the obtained purified enzyme was subjected to isoelectric focusing by the method shown in 6. The obtained purified enzyme was subjected to SDS-PAGE that employs gel PAG Mini DAIICHI 7.5 (Daiichi Kagaku Yakuhin). A transfer buffer solution 1 (0.3M Tris (pH 10.4), 20% methanol), a transfer buffer solution 2 (30 mM Tris (pH 10.4), 20% methanol), and a transfer buffer solution 3 (40 mM 6-aminohexanoic acid (pH 7.6), 20% methanol) were used as a transfer buffer solution, and thus obtained gel was transferred to a PVDF membrane. Transfer operation was carried out by using a semi-dry transfer device at constant current of 0.8 mA/PVDF membrane $cm^2$ for 90 min in which the transfer buffer solution 1 was disposed at the anode side, the transfer buffer solution 2 was disposed in a central part including membrane, and the transfer buffer solution 3 was disposed at the cathode side. After transfer, CBB (Coomassie Brilliant Blue R-250) staining was carried out and a band corresponding to the enzyme was cut out to obtain a sample for analyzing the N-terminal amino acid sequence. Similarly, thus obtained gel was subjected to CBB staining and a band corresponding to the enzyme was cut out to obtain a sample for analyzing the internal amino acid sequence. As a result of analysis, the N-terminal amino acid sequence and the internal amino acid sequence were clarified.

(2) Homology Search by N-terninal amino acid sequence

Among the gene information of Aspergillus oryzae RIB40 disclosed in Japanese Patent Application Unexamined Publication No. 2005-176602, entire CDS sequence was obtained from the applicant of this patent application, and homology search of the clarified N-terminal amino acid sequence was carried out by using Database Software Kiroku Ver. 3 (World Fusion Co., Ltd.). As a result, high homology with respect to the amino acid sequence homologous to Glucose Oxidase Precursor (SEQ ID NO: 20494 in the sequence list in Japanese Patent Application Unexamined Publication No. 2005-176602) was observed. The sequence also includes a region corresponding to the amino acid sequence that is clarified by the analysis of the internal amino acid sequence. From these facts, it is clear that this sequence (SEQ ID NO: 1) corresponds to the amino acid sequence of the enzyme.

(3) Extraction of genome from Aspergillus oryzae BB-56

Aspergillus oryzae BB-56 was cultured in a shaking flask including 100 ml of YPD medium (1% Yeast Extract, 2% Peptone, 2% Glucose) at 30°C over night. Then, the culture solution was filtrated by using a Buchner funnel and Nutsche suction bottle so as to obtain a fungus body, which was frozen at -80°C and freeze-dried. After drying, about 0.3 g of the obtained fungus body was pulverized together with a spoonful of sea sand in a mortar with a pestle. The pulverized fungus body was suspended in 12 ml of extraction buffer (1% hexadecyltrimethylammonium bromide, 0.7M NaCl, 50 mM Tris-HCl (pH 8.0), 10 mM EDTA, 1% mercaptoethanol) and stirred at room temperature for 30 minutes. Then, equal amount of phenol : chloroform : isoamyl alcohol (25 : 24 : 1) solution was added, stirred and centrifuged (1,500g, 5 minutes, room temperature) so as to obtain supernatant. An equal amount of isopropanol was gently added to the resultant supernatant. Chromosome DNA precipitated with this treatment was centrifuged (20,000g, 10 minutes, 4°C) so as to obtain precipitate. The precipitate was washed with 70% ethanol and vacuum-dried. The thus obtained chromosome DNA was dissolved in 4 ml of TE again. 200 μl of 10 mg/ml RNase A (SIGMA-ALDRICH Japan K.K.) was added and incubated at 37°C for 30 minutes. Then, 40 μl of 20 mg/ml Proteinase K, recombinant, PCR Grade (Roche Diagnostics K.K.) solution was added and incubated at 37°C for 30 minutes. Then, an equal amount of phenol : chloroform : isoamyl alcohol (25 : 24 : 1) solution was added and stirred. Thereafter, it was centrifuged (1,500g, 5 minutes, room temperature) so as to obtain a supernatant. This washing operation was repeated twice. Then, an equal amount of chloroform : isoamyl alcohol (24 : 1) solution was added, stirred and then centrifuged (1,500g, 5 minutes, room temperature). 1/10 volume of 3M NaOAc (pH 4.8) and twice volume of ethanol were added to the resultant supernatant and cooled at -80°C, and thereby chromosome DNA was precipitated. The precipitated chromosome DNA was recovered by centrifugation (20,000g, 20 minutes, 4°C). The recovered chromosome DNA was washed with 70% ethanol, then vacuum-dried, and finally dissolved in 400 μl of TE solution. Thus, chromosome DNA solution whose concentration was about 1 mg/ml was obtained.

(4) Design of Synthesized Primer

Synthesized primers FG-F and FG-R (SEQ ID NO: 3 and 4) corresponding to the sequences at 5'-, and 3'-terminals corresponding of the gene sequence (SEQ ID NO: 2) derived from RIB40 strain having a full chain length of 3226 bp, which encodes the amino acid sequence set forth in SEQ ID NO: 1 (sequence set forth in SEQ ID NO: 20494 in Japanese Patent Application Unexamined Publication No. 2005-176602) were synthesized.

(5) Amplification of FAD-GDH gene by PCR

PCR reaction using the chromosome DNA prepared in (3) as a template was carried out. The reaction was carried out by using TAKARA LA-Taq™ (TAKARA BIO INC.), and the composition of the reaction solution was determined by the attached conventional method. PCR reaction was carried out in which 0.4 μM each of primers (FG-F and FG-R) were added so that the concentration of the template genome was 10 ng/μl. The reaction cycle included reaction at 94°C for 2 minutes, reaction at 94°C for 30 seconds, reaction at 60°C for 30 seconds, and reaction at 72°C for 10 minutes. The cycle was repeated 35 times. Finally, the reaction at 72°C for 10 minutes was carried out. After reaction, the sample was stored at 4°C.

(6) Analysis of Base Sequence of FAD-GDH Gene

The PCR reaction solution was subjected to agarose electrophoresis so as to separate into an amplification fragment and a primer and extracted from agarose gel by using GENECLEANT™ III (BIO 101). The extracted amplification fragment was sub-cloned to the SmaI site of the vector pUC19 (TAKARA BIO INC.). Then, the base sequence of the region corresponding to the enzyme gene in the sub-cloned plasmid pFG2 was analyzed. The sequence reaction was carried out by using BigDye™ Terminator v3.1 Cycle Sequencing Kit (Applied Biosystems Japan) and according to the instruction of the product. For analysis, ABI PRISM 310 sequencer (Applied Biosystems Japan) was used. In order to analyze the base sequence of the enzyme gene, synthesized primers: FG-1 (SEQ ID NO: 5), FG-2 (SEQ ID NO: 6), FG-3 (SEQ ID NO: 7), FG-4 (SEQ ID NO: 8), FG-5 (SEQ ID NO: 9), FG-6 (SEQ ID NO: 10), FG-7 (SEQ ID NO: 11), FG-8 (SEQ ID NO: 12), FG-9 (SEQ ID NO: 13), FG-10 (SEQ ID NO: 14), FG-11 (SEQ ID NO: 15), FG-12 (SEQ ID NO: 16), M13-M5 (SEQ ID NO: 17), and M13-RV2 (SEQ ID NO: 18) were synthesized. As a result of the analysis of the base sequence, a gene sequence set forth in SEQ ID NO: 19 having full length of 3241bp of the enzyme derived from Aspergillus oryzae BB-56 was clarified. The amino acid sequence of the enzyme gene predicted from the gene sequence is shown in SEQ ID NO: 20.

[Industrial Applicability]

FAD-GDH of the present invention is excellent in the substrate specificity and allows more accurate measurement of the glucose amount. Therefore, the FAD-GDH of the present invention is suitable for measuring the blood sugar level or measuring the glucose level in foods (seasoning agents and beverage).

## Claims

1. A purified flavin adenine dinucleotide-binding glucose dehydrogenase consisting of the amino acid sequence set forth in SEQ ID NO: 20, wherein the flavin adenine dinucleotide-binding glucose dehydrogenase comprises the following properties:

   (1) action: catalyzing a reaction of oxidizing a hydroxyl group of glucose in a presence of an electron acceptor to yield glucono-δ-lactone;
   (2) molecular weight: about 100 kDa when measured by SDS-polyacrylamide electrophoresis and about 400 kDa when measured by gel filtration chromatography;
   (3) substrate specificity: having a reactivity with respect to maltose, D-fructose, D-mannose and D-galactose of 5% or less when the reactivity with respect to D-glucose is assumed to be 100%;
   (4) Sugar content: having a sugar content of about 43% (w/w).

2. The purified flavin adenine dinucleotide-binding glucose dehydrogenase according to claim 1, further comprising the following properties:

   (5) optimum pH: about 7;
   (6) optimum temperature: about 60°C;
   (7) pH for stability: stable in the range from pH 3.0 to pH 7.0; and
   (8) temperature for stability: stable at 40°C or less.

3. The purified flavin adenine dinucleotide-binding glucose dehydrogenase according to claim 1 or 2, further comprising the following property:

   (9) Km value: about 8 mM with respect to D-glucose.

4. The purified flavin adenine dinucleotide-binding glucose dehydrogenase according to any one of claims 1 to 3, which is derived from Aspergillus oryzae.

5. Aspergillus oryzae BB-56 deposited under the accession number of NITE BP-236, which has an ability to produce flavin adenine dinucleotide-binding glucose dehydrogenase.

6. A flavin adenine dinucleotide-binding glucose dehydrogenase gene encoding the purified flavin adenine dinucleotide-binding glucose dehydrogenase according to any one of claims 1 to 4, and consisting of the base sequence set forth in SEQ ID NO: 19.

**7.** A vector containing the gene according to claim 6.

**8.** A non-human transformant in which the gene according to claim 6 is introduced.

**9.** A method for manufacturing flavin adenine dinucleotide-binding glucose dehydrogenase according to any one of claims 1 to 4, the method comprising the following steps (1) and (2):

(1) culturing Aspergillus oryzae having an ability to produce the flavin adenine dinucleotide-binding glucose dehydrogenase according to claim 4;
(2) recovering the flavin adenine dinucleotide-binding glucose dehydrogenase from a culture solution and/or a fungus body after culturing by purification comprising the steps as set forth in Table 2.

**10.** A method for manufacturing flavin adenine dinucleotide-binding glucose dehydrogenase according to any one of claims 1 to 4, the method comprising the following steps (i) and (ii):

(i) culturing a transformant according to claim 8 under a condition in which protein encoded by the gene is produced; and
(ii) recovering the produced protein.

**11.** A method for measuring glucose, wherein glucose in a sample is measured by using the flavin adenine dinucleotide-binding glucose dehydrogenase according to any one of claims 1 to 4.

**12.** A reagent for measuring glucose, comprising the purified flavin adenine dinucleotide-binding glucose dehydrogenase according to any one of claims 1 to 4.

**13.** A kit for measuring glucose comprising the reagent for measuring glucose according to claim 12.

**Patentansprüche**

**1.** Gereinigte flavin-adenin-dinukleotid bindende Glukosedehydrogenase bestehend aus der Aminosäuresequenz dargelegt in SEQ ID Nr.: 20; wobei die flavin-adenin-dinukleotid bindende Glukosedehydrogenase die folgenden Eigenschaften umfasst:

(1) Wirkung: Katalysieren einer Reaktion zur Oxidation einer Hydroxylgruppe von Glucose in Anwesenheit eines Elektronenakzeptor um Glucono-δ-Lacton zu erzeugen;
(2) Molekulargewicht: etwa 100 kDa wenn durch eine SDS-Polyacrylamid Elektrophorese gemessen und etwa 400 kDa wenn durch eine Gelfiltrationschromatographie gemessen;
(3) Substratspezifität: weist eine Reaktivität von 5% oder weniger gegenüber Maltose, D-Fructose, D-Mannose und D-Galaktose auf wenn die Reaktivität in Bezug zu D-Glukose als 100% angenommen wird;
(4) Zuckergehalt: weist einen Zuckergehalt von etwa 43% (w/w) auf.

**2.** Gereinigte flavin-adenin-dinukleotid bindende Glukosedehydrogenase nach Anspruch 1, weiterhin die folgende Eigenschaft umfassend:

(5) Optimaler pH: etwa 7;
(6) Optimale Temperatur: etwa 60°C;
(7) pH Wert für Stabilität: stabil in einem Bereich von pH 3.0 bis zu pH 7.0; und
(8) Temperaturstabilität: stabil bei 40°C oder weniger.

**3.** Gereinigte flavin-adenin-dinukleotid bindende Glukosedehydrogenase nach Anspruch 1 oder 2, weiterhin die folgende Eigenschaft umfassend:

(9) Km Wert: ungefähr 8 mM in Bezug zu D-Glukose.

4. Gereinigte flavin-adenin-dinukleotid bindende Glukosedehydrogenase nach einem der Ansprüche 1 bis 3, welche aus Aspergillus oryzae gewonnen wird.

5. Aspergillus oryzae BB-56 hinterlegt unter der Zugangsnummer NITE BP-236, welches die Fähigkeit hat flavin-adenin-dinukleotid bindende Glukosedehydrogenase herzustellen.

6. Flavin-adenin-dinukleotid bindendes Glukosedehydrogenase Gen kodierend für die gereinigte flavin-adenin-dinukleotid bindende Glukosedehydrogenase nach einem der Ansprüche 1 bis 4, und bestehend aus der Basensequenz dargelegt in SEQ ID Nr.: 19.

7. Vektor, umfassend das Gen nach Anspruch 6.

8. Nicht-humane Transformante in welche das Gen nach Anspruch 6 eingebracht ist.

9. Verfahren zur Herstellung von flavin-adenin-dinukleotid bindenden Glukosedehydrogenase nach einem der Ansprüche 1 bis 4, das Verfahren umfassend die folgenden Schritte (1) und (2):

(1) Kultivieren von Aspergillus oryzae welches die Fähigkeit aufweist die flavin-adenin-dinukleotid bindende Glukosedehydrogenase nach Anspruch 4 zu produzieren;
(2) Gewinnen der flavin-adenin-dinukleotid bindende Glukosedehydrogenase aus einer Kulturlösung und/oder eines Pilzkörpers nach Gewinnung durch Aufreinigung umfassend die Schritte wie in Tabelle 2 aufgeführt.

10. Verfahren zur Herstellung von flavin-adenin-dinukleotid bindende Glukosedehydrogenase nach einem der Ansprüche 1 bis 4, das Verfahren umfassend die folgenden Schritte (i) und (ii):

(i) Kultivieren einer Transformante nach Anspruch 8 unter solchen Bedingungen, dass das Protein, kodiert durch das Gen, produziert wird; und
(ii) Gewinnen des hergestellten Proteins.

11. Verfahren zur Messung von Glukose, wobei Glukose in einer Probe durch Verwendung der flavin-adenin-dinukleotid bindenden Glukosedehydrogenase nach einem der Ansprüche 1 bis 4 gemessen wird.

12. Reagenz zur Messung von Glukose, umfassend die gereingte flavin-adenin-dinukleotid bindende Glukosedehydrogenase nach einem der Ansprüche 1 bis 4.

13. Kit zur Messung von Glukose, umfassend das Reagenz zur Messung von Glukose nach Anspruch 12.

## Revendications

1. Glucose déshydrogénase purifiée se liant au dinucléotide flavine-adénine constituée par la séquence d'acides aminés exposée dans SEQ ID N: 20, dans laquelle la glucose déshydrogénase se liant au dinucléotide flavine-adénine possède les propriétés suivantes:

(1) action : catalyse une réaction d'oxydation d'un groupe hydroxyle du glucose en présence d'un accepteur d'électrons pour donner une glucono-δ-lactone;
(2) poids moléculaire : environ 100 kDa, quand il est mesuré par électrophorèse sur SDS-polyacrylamide et environ 400 kDa, quand il est mesuré par chromatographie par filtration sur gel;
(3) spécificité de substrat : manifeste une réactivité vis-à-vis du maltose, du D-fructose, du D-mannose et du D-galactose de 5 % ou moins quand sa réactivité vis-à-vis du D-glucose est supposée être de 100 %;
(4) teneur en sucre : a une teneur en sucre d'environ 43 % (p/p).

2. Glucose déshydrogénase purifiée se liant au dinucléotide flavine-adénine selon la revendication 1 ayant, en outre, les propriétés suivantes :

(5) pH optimal: environ 7;
(6) température optimale: environ 60 °C;
(7) pH de stabilité: stable dans la plage de pH 3,0 à pH 7,0; et

(8) température de stabilité: stable à 40 °C ou moins.

**3.** Glucose déshydrogénase purifiée se liant au dinucléotide flavine-adénine selon les revendications 1 ou 2 ayant, en outre, la propriété suivante:

(9) valeur Km: environ 8 mM par rapport au D-glucose.

**4.** Glucose déshydrogénase purifiée se liant au dinucléotide flavine-adénine selon l'une quelconque des revendications 1 à 3, qui est dérivée d'*Aspergillus oryzae*.

**5.** *Aspergillus oryzae* BB-56 déposé sous le numéro d'accès de NITE BP-236, ayant la capacité de produire une glucose déshydrogénase se liant au dinucléotide flavine-adénine.

**6.** Gène de la glucose déshydrogénase se liant au dinucléotide flavine-adénine codant pour la glucose déshydrogénase purifiée se liant au dinucléotide flavine-adénine selon l'une quelconque des revendications 1 à 4, et se composant de la séquence de bases exposée dans SEQ ID N: 19.

**7.** Vecteur contenant le gène selon la revendication 6.

**8.** Transformant non humain chez lequel le gène selon la revendication 6 est introduit.

**9.** Procédé de préparation de la glucose déshydrogénase se liant au dinucléotide flavine-adénine selon l'une quelconque des revendications 1 à 4, le procédé comprenant les étapes (1) et (2) suivantes:

(1) culture d'*Aspergillus oryzae* ayant la capacité de produire la glucose déshydrogénase se liant au dinucléotide flavine-adénine selon la revendication 4;
(2) récupération de la glucose déshydrogénase se liant au dinucléotide flavine-adénine à partir d'une solution de culture et/ou d'un corps fongique après culture, par un procédé de purification comprenant les étapes exposées dans le Tableau 2.

**10.** Procédé de préparation de la glucose déshydrogénase se liant au dinucléotide flavine-adénine selon l'une quelconque des revendications 1 à 4, le procédé comprenant les étapes (i) et (ii) suivantes:

(i) culture d'un transformé selon la revendication 8 dans une condition dans laquelle une protéine codée par le gène est produite; et
(ii) récupération de la protéine produite.

**11.** Procédé de mesure du glucose, dans lequel le glucose présent dans un échantillon est mesuré à l'aide de la glucose déshydrogénase se liant au dinucléotide flavine-adénine selon l'une quelconque des revendications 1 à 4.

**12.** Réactif de mesure du glucose, comprenant la glucose déshydrogénase se liant au dinucléotide flavine-adénine purifiée selon l'une quelconque des revendications 1 à 4.

**13.** Kit de mesure du glucose comprenant le réactif de mesure du glucose selon la revendication 12.

Fig.1

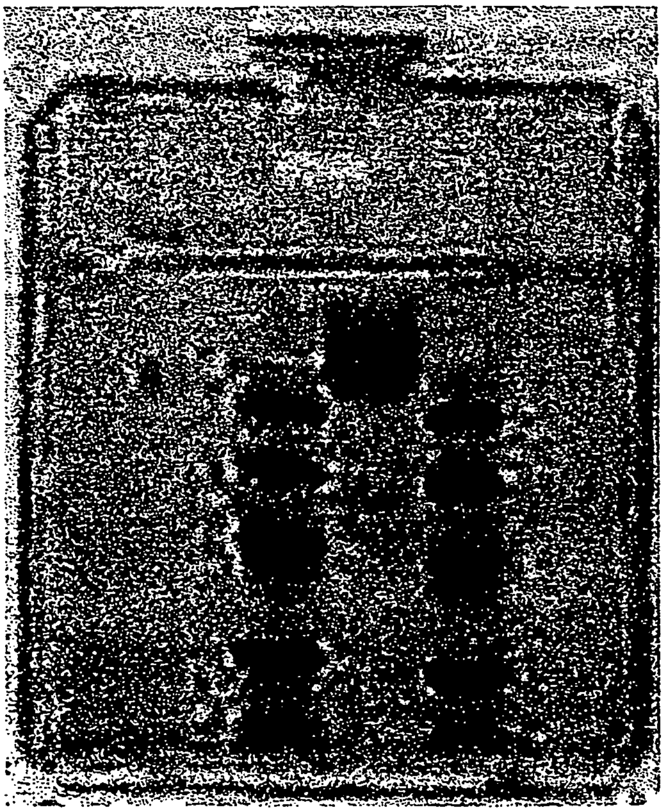

Fig.2

(A)

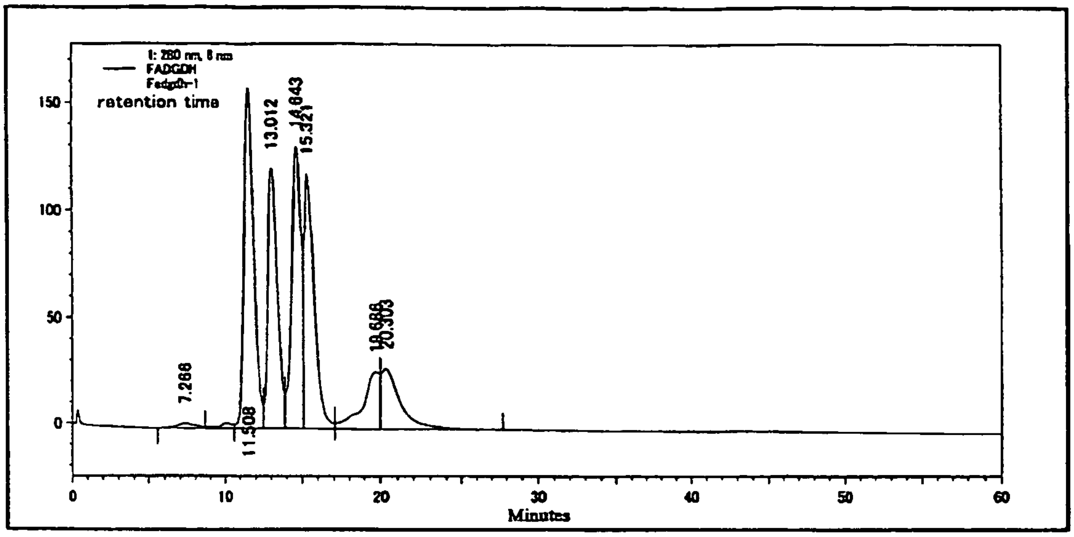
1: 280 nm, 8 nm
FADGDH
retention time
150
100
50
0
7.266
11.508
13.012
14.843
15.321
19.666
20.303
0
10
20
30
40
50
60
Minutes

(B)

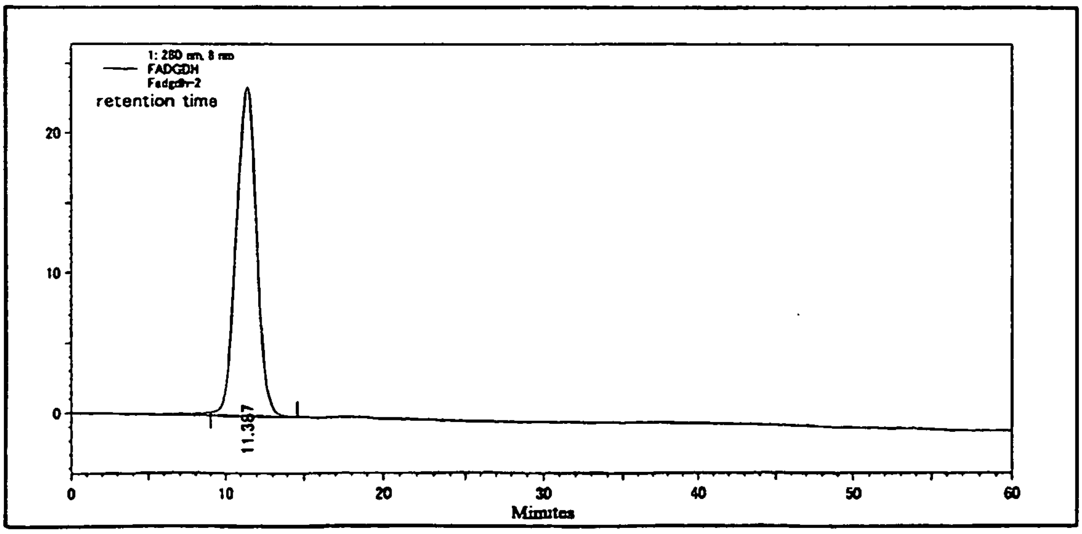
1: 280 nm, 8 nm
FADGDH
retention time
20
10
0
11.387
0
10
20
30
40
50
60
Minutes

Fig.3

(A)

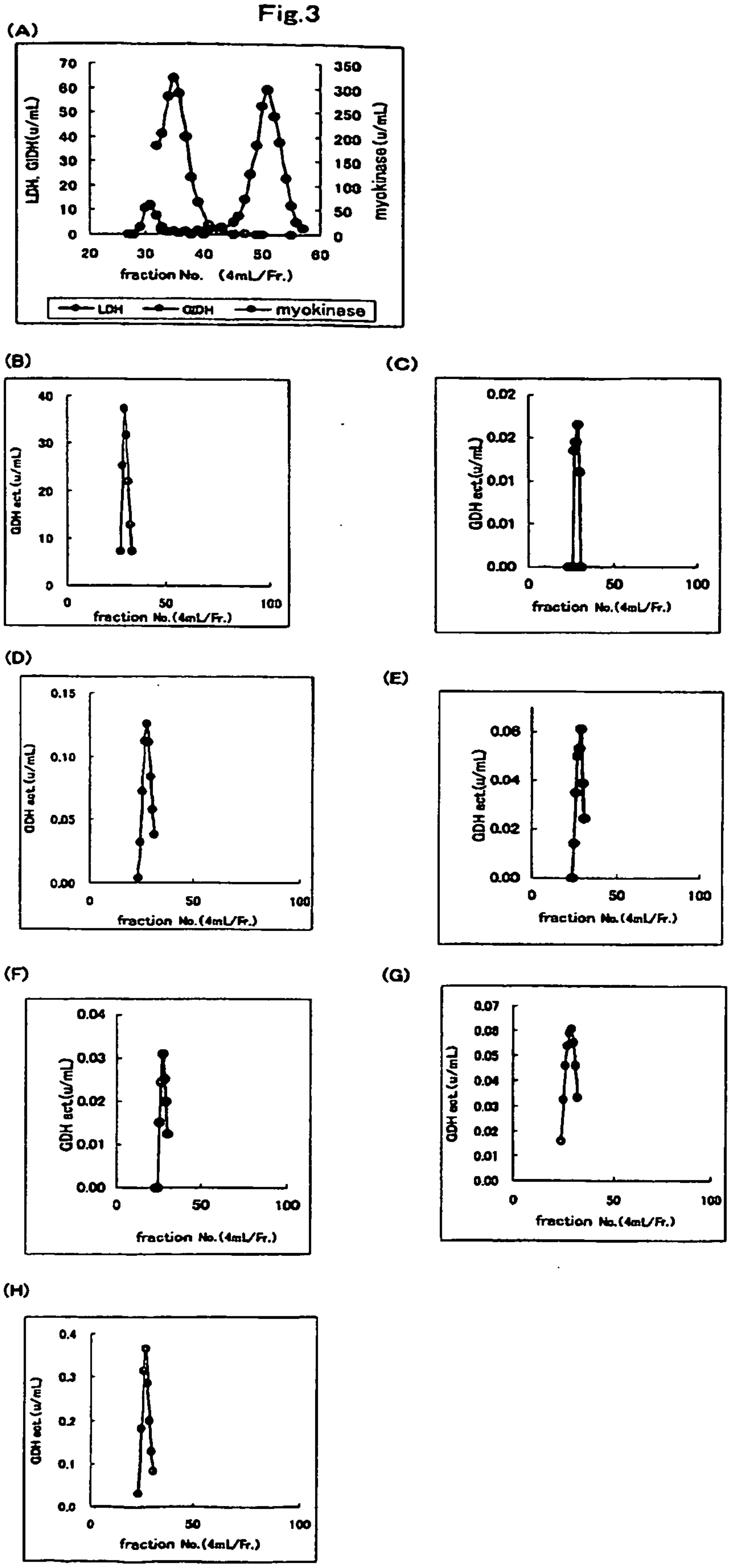
LDH, GlDH(u/mL)
myokinase(u/mL)
fraction No. (4mL/Fr.)
LDH
GlDH
myokinase
(B)
GDH act.(u/mL)
fraction No.(4mL/Fr.)
(C)
GDH act.(u/mL)
fraction No.(4mL/Fr.)
(D)
GDH act.(u/mL)
fraction No.(4mL/Fr.)
(E)
GDH act.(u/mL)
fraction No.(4mL/Fr.)
(F)
GDH act.(u/mL)
fraction No.(4mL/Fr.)
(G)
GDH act.(u/mL)
fraction No.(4mL/Fr.)
(H)
GDH act.(u/mL)
fraction No.(4mL/Fr.)

Fig.4

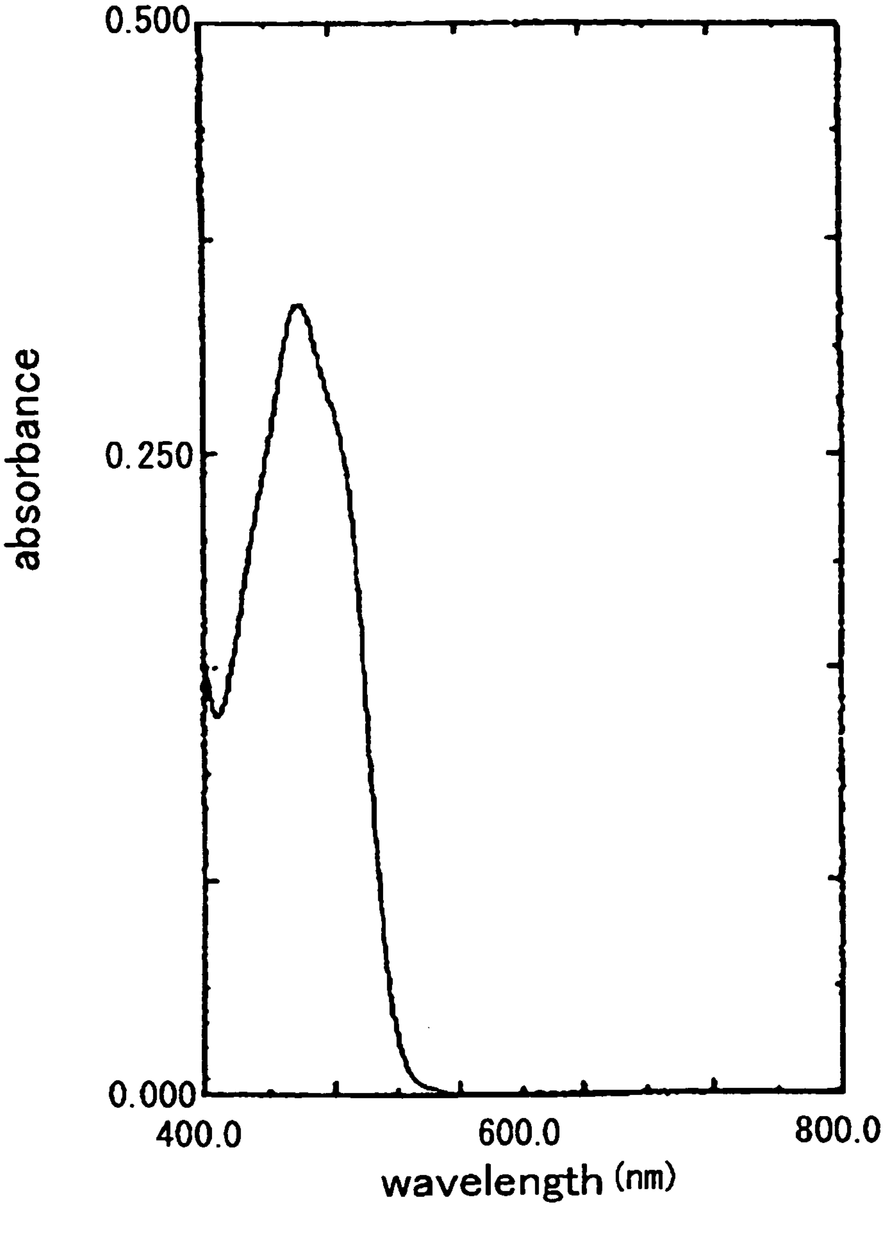

Fig.5

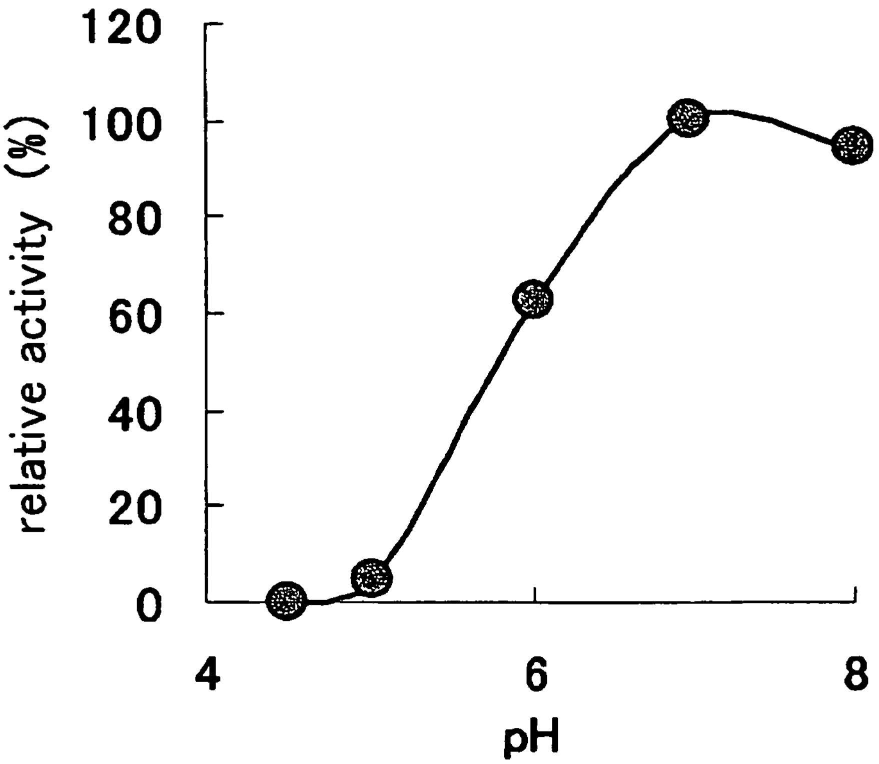

Fig.6

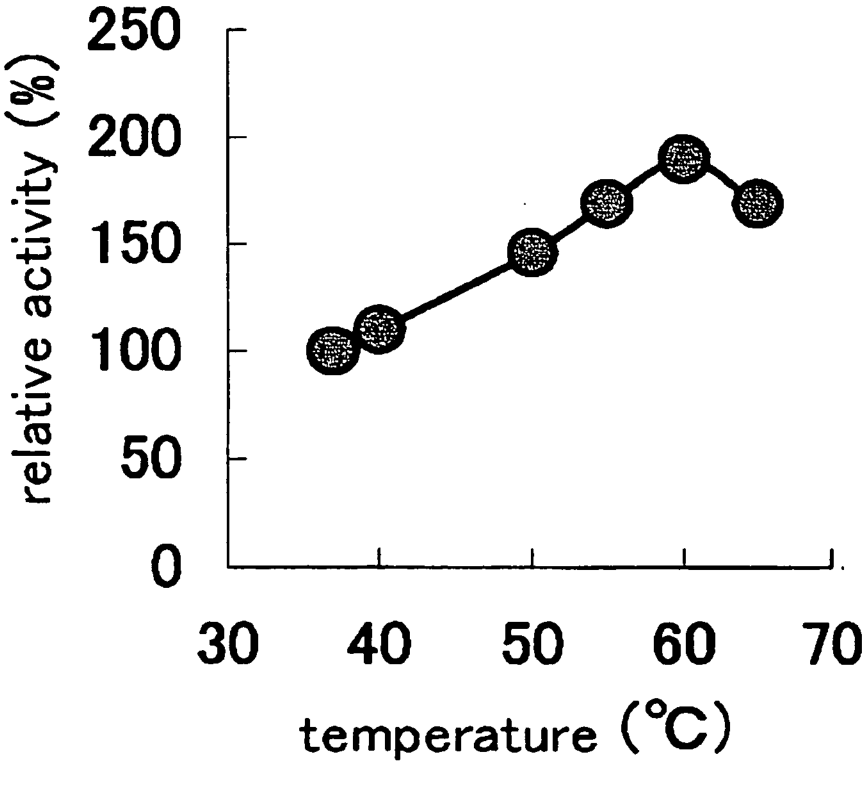
250
200
150
100
50
0
relative activity (%)
30
40
50
60
70
temperature (°C)

Fig.7

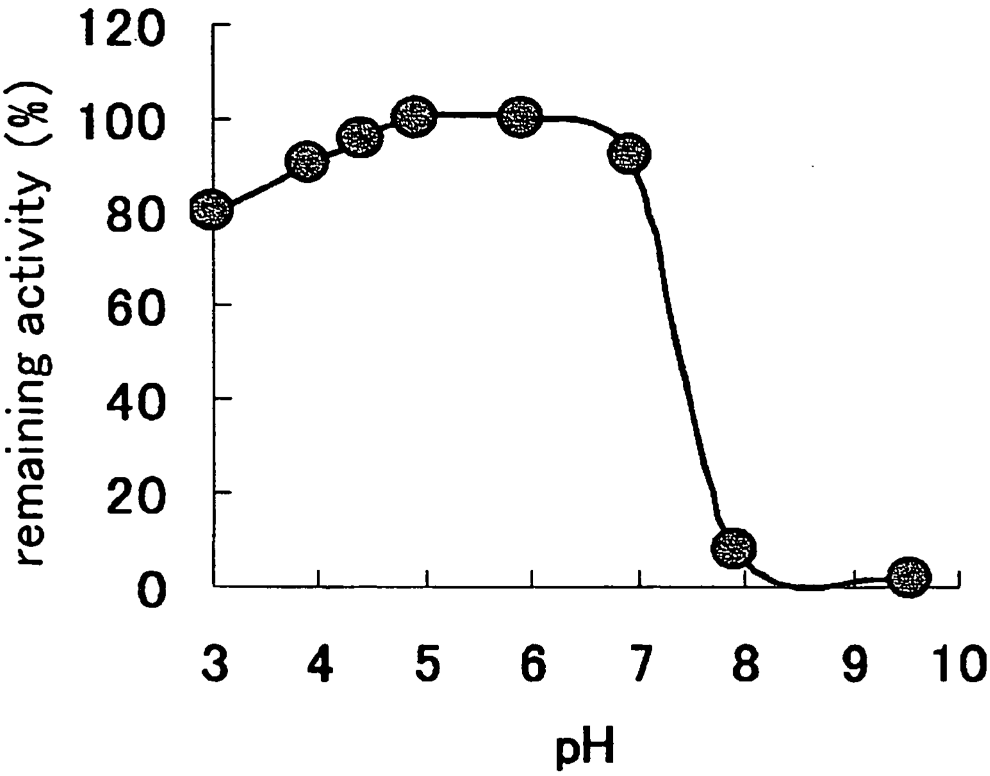

Fig.8

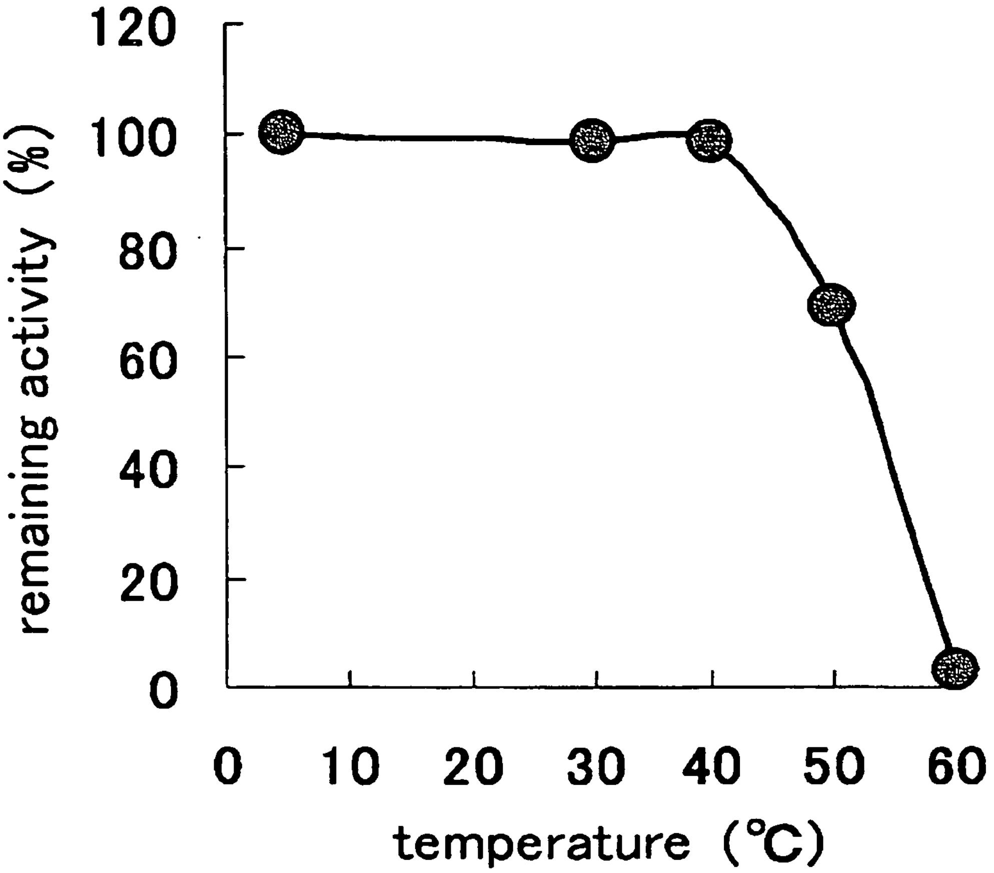
120
100
80
60
40
20
0
remaining activity (%)
0
10
20
30
40
50
60
temperature (℃)

Fig.9

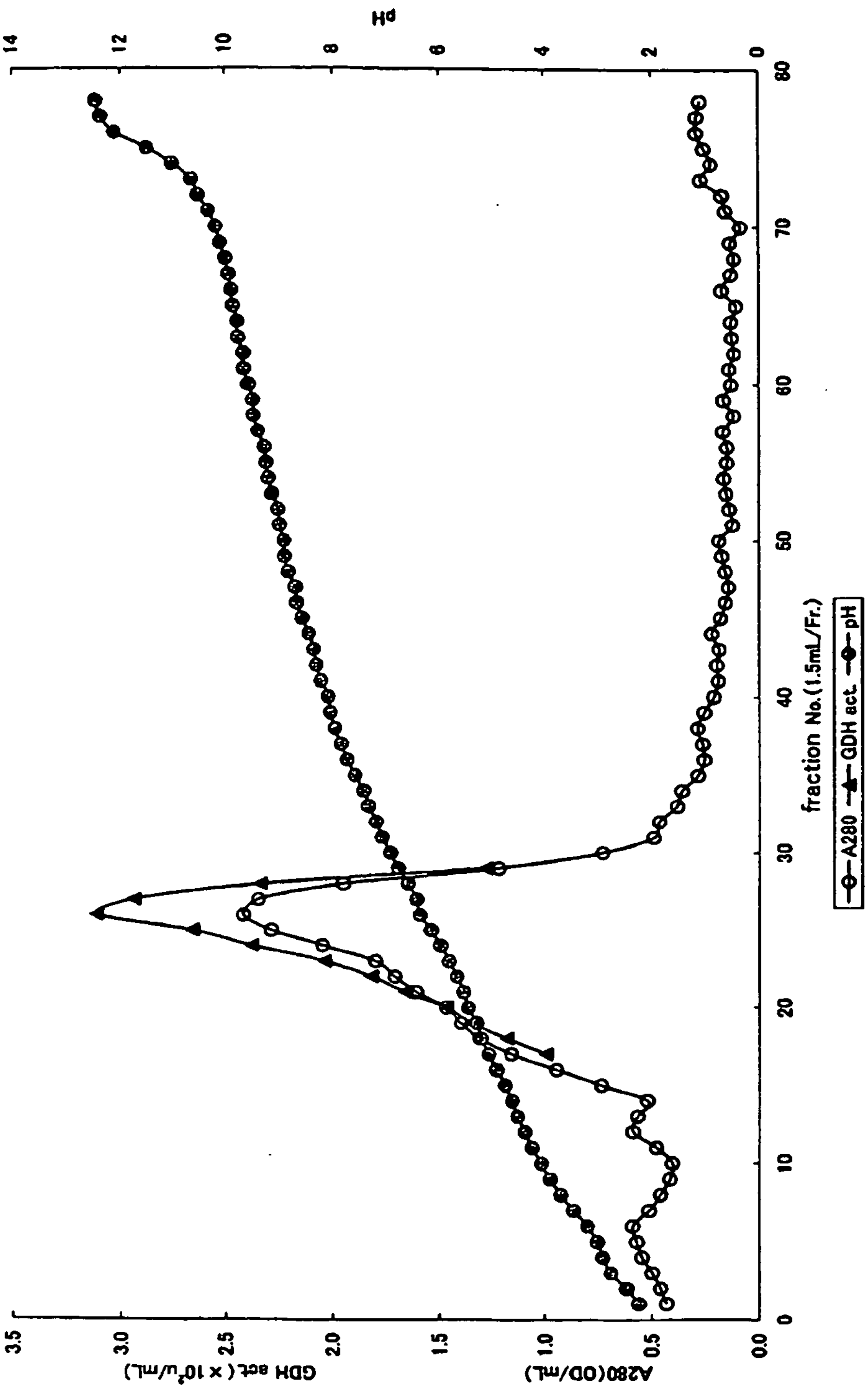
GDH act ( x 10²u/mL)
A280(OD/mL)
pH
fraction No.(1.5mL/Fr.)
A280
GDH act
pH
0.0
0.5
1.0
1.5
2.0
2.5
3.0
3.5
0
10
20
30
40
50
60
70
80
0
2
4
6
8
10
12
14

# Fig.10

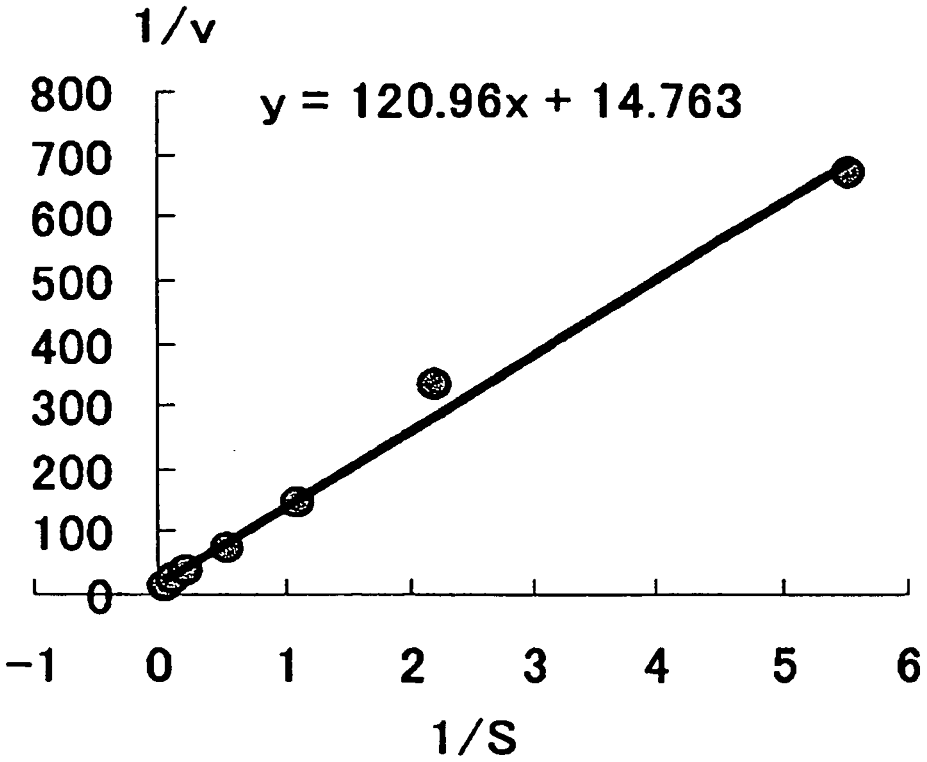

Fig.11

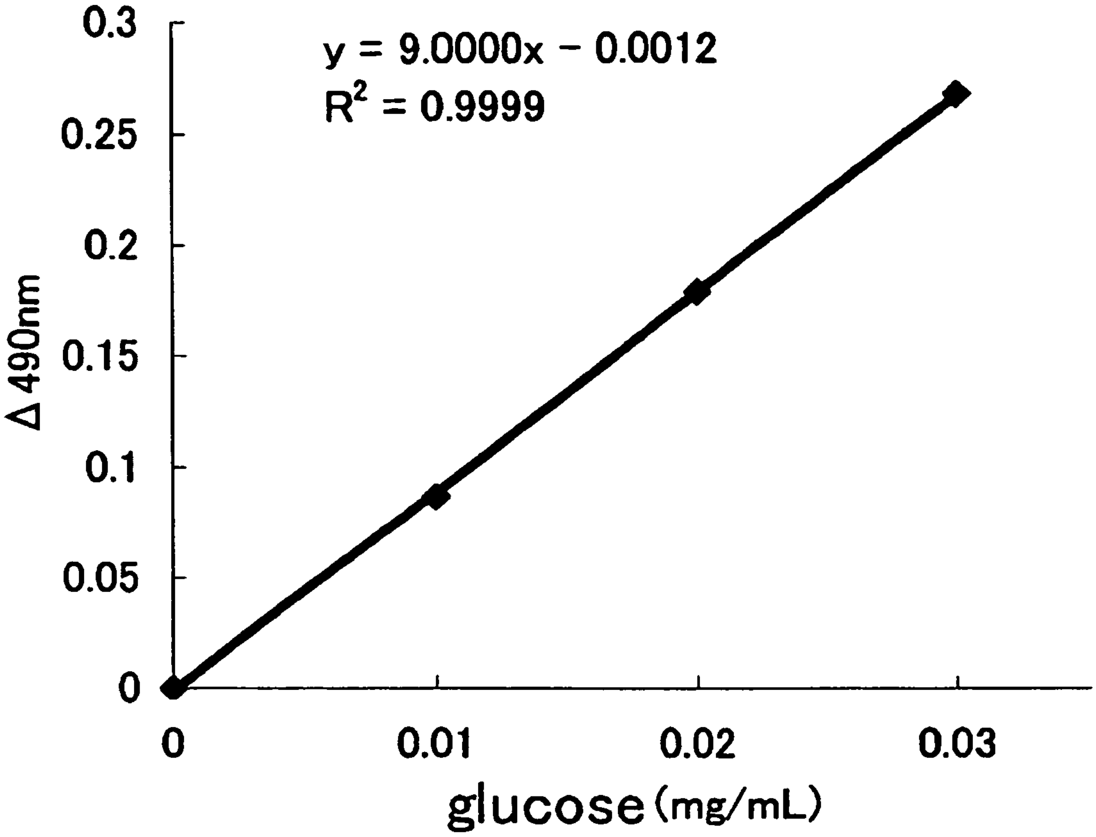
y = 9.0000x - 0.0012
R2 = 0.9999
0.3
0.25
0.2
0.15
0.1
0.05
0
Δ490nm
0
0.01
0.02
0.03
glucose (mg/mL)

Fig.12

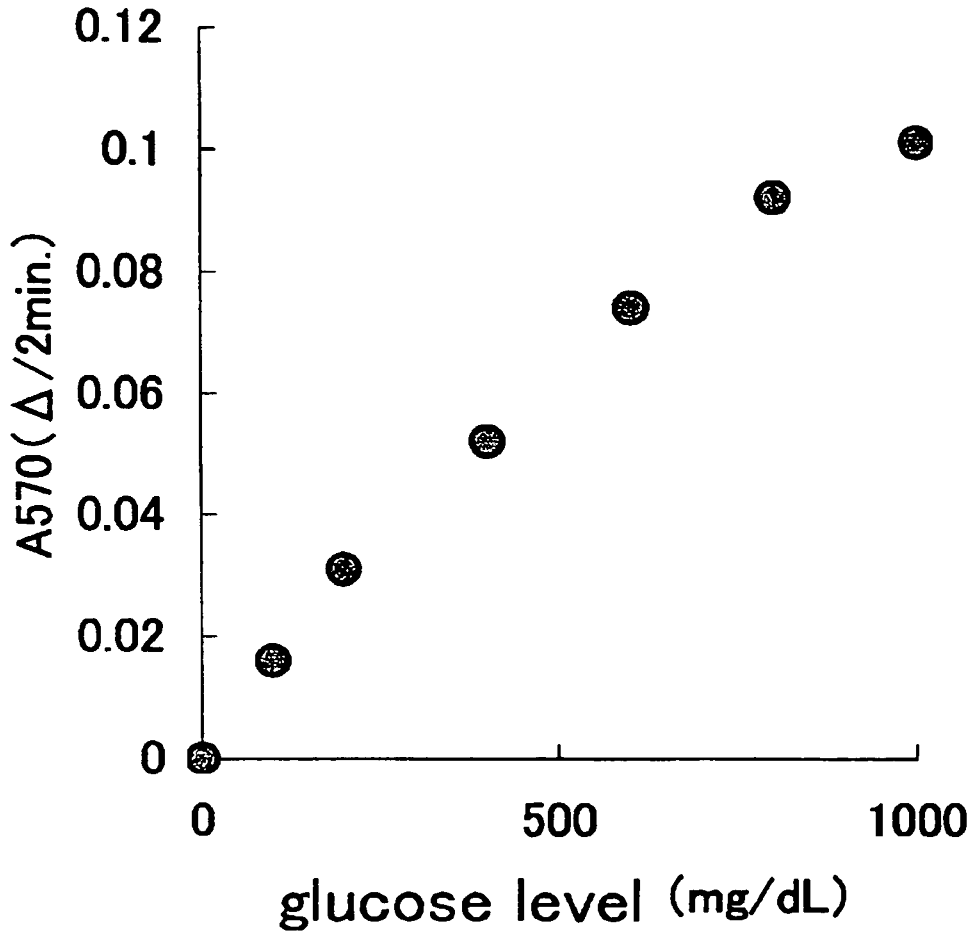
0.12
0.1
0.08
0.06
0.04
0.02
0
A570(Δ/2min.)
0
500
1000
glucose level (mg/dL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000350588 A
- JP 2001197888 A
- JP 2001346587 A
- WO 2004058958 A
- JP 2005176602 A
- JP P2006148667 B


### Non-patent literature cited in the description


- **T.C. Bak ; R. Sato.** *Biochim. Biophys. Acta,* 1967, vol. 139, 265-276
- **T.C. Bak.** *Biochim. Biophys. Acta,* 1967, vol. 139, 277-293
- **T.C. Bak.** *Biochim. Biophys. Acta,* 1967, vol. 146, 317-327
- **T.C. Bak ; R. Sato.** *Biochim. Biophys. Acta,* 1967, vol. 146, 328-335
- **Karlin ; Altschul.** *Proc. Natl. Acad. Sci. USA,* 1990, vol. 87, 2264-68
- **Karlin ; Altschul.** *Proc. Natl. Acad. Sci. USA,* 1993, vol. 90, 5873-77
- **Altschul et al.** *Amino Acids Research,* 1997, vol. 25 (17), 3389-3402
- **Myers ; Miller.** *Comput Appl Biosci.,* 1988, vol. 4, 11-17
- Molecular Cloning. Cold Spring Harbor Laboratory Press
- Current protocols in molecular biology. 1987
- Molecular Cloning. Spring Harbor Laboratory Press
- **Potter, H. et al.** *Proc. Natl. Acad. Sci. U.S.A.,* 1984, vol. 81, 7161-7165
- **Felgner, P.L. et al.** *Proc. Natl. Acad. Sci. U.S.A.,* 1984, vol. 84, 7413-7417
- **Graessmann, M. ; Graessmann, A.** *Proc. Natl. Acad. Sci. U.S.A.,* 1976, vol. 73, 366-370
- **Hanahan, D.** *J. Mol. Biol.,* 1983, vol. 166, 557-580
- **Schiestl, R.H. et al.** *Curr. Genet.,* 1989, vol. 16, 339-346
- **Yelton, M.M. et al.** *Proc. Natl. Acad. Sci.,* 1984, vol. 81, 1470-1474
- **Raper, K. B. ; Fennell, D. I.** The genus Aspergillus. Williams & Wilkins Co, 1965, 686 pp
- **Kozakiewicz, Z.** Aspergillus species on stored products. *Mycological Papers.,* 2002, 1-188
- Identification of Common Aspergillus species. *Utrecht : Centraalbureau voor Schimmelcultures,* 116 pp
- **Samson, R. A. Hoekstra ; E. S. Frisvad ; J. C. ; Filtenborg, O.** Introduction to food- and airborne fungi. 64-97

SEQUENCE LISTING

<110> AMANO ENZYME INC.
TANAKA, Noriaki
YUUKI, Kensuke

<120> FLAVIN ADENINE DINUCLEOTIDE-BINDING GLUCOSE DEHYDROGENASE

<130> P06045P

<150> JP P2006-148667 <151> 2006-05-29

<160> 20

<170> PatentIn version 3.4

<210> 1
<211> 588
<212> PRT
<213> Aspergillus oryzae

<400> 1

```
Met Leu Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val
            20                  25                  30

Val Gly Gly Gly Thr Ser Gly Leu Val Val Ala Asn Arg Leu Ser Glu
        35                  40                  45

Asn Pro Asp Val Ser Val Leu Leu Leu Glu Ala Gly Ala Ser Val Phe
    50                  55                  60

Asn Asn Pro Asp Val Thr Asn Ala Asn Gly Tyr Gly Leu Ala Phe Gly
65                  70                  75                  80

Ser Ala Ile Asp Trp Gln Tyr Gln Ser Ile Asn Gln Ser Tyr Ala Gly
                85                  90                  95

Gly Lys Gln Gln Val Leu Arg Ala Gly Lys Ala Leu Gly Gly Thr Ser
            100                 105                 110

Thr Ile Asn Gly Met Ala Tyr Thr Arg Ala Glu Asp Val Gln Ile Asp
        115                 120                 125

Val Trp Gln Lys Leu Gly Asn Glu Gly Trp Thr Trp Lys Asp Leu Leu
    130                 135                 140

Pro Tyr Tyr Leu Lys Ser Glu Asn Leu Thr Ala Pro Thr Ser Ser Gln
```

145 150 155 160

Val Ala Ala Gly Ala Ala Tyr Asn Pro Ala Val Asn Gly Lys Glu Gly
165 170 175

Pro Leu Lys Val Gly Trp Ser Gly Ser Leu Ala Ser Gly Asn Leu Ser
180 185 190

Val Ala Leu Asn Arg Thr Phe Gln Ala Met Glu Asp Val Asn Gly Gly
195 200 205

Lys Met Arg Gly Phe Asn Ile Tyr Pro Ser Thr Leu Asp Val Asp Leu
210 215 220

Asn Val Arg Glu Asp Ala Ala Arg Ala Tyr Tyr Phe Pro Tyr Asp Asp
225 230 235 240

Arg Lys Asn Leu His Leu Leu Glu Asn Thr Thr Ala Asn Arg Leu Phe
245 250 255

Trp Lys Asn Gly Ser Ala Glu Glu Ala Ile Ala Asp Gly Val Glu Ile
260 265 270

Thr Ser Ala Asp Gly Lys Val Thr Arg Val His Ala Lys Lys Glu Val
275 280 285

Ile Ile Ser Ala Gly Ala Leu Arg Ser Pro Leu Ile Leu Glu Leu Ser
290 295 300

Gly Val Gly Asn Pro Thr Ile Leu Lys Lys Asn Asn Ile Thr Pro Arg
305 310 315 320

Val Asp Leu Pro Thr Val Gly Glu Asn Leu Gln Asp Gln Phe Asn Asn
325 330 335

Gly Met Ala Gly Glu Gly Tyr Gly Val Leu Ala Gly Ala Ser Thr Val
340 345 350

Thr Tyr Pro Ser Ile Ser Asp Val Phe Gly Asn Glu Thr Asp Ser Ile
355 360 365

Val Ala Ser Leu Arg Ser Gln Leu Ser Asp Tyr Ala Ala Ala Thr Val
370 375 380

Lys Val Ser Asn Gly His Met Lys Gln Glu Asp Leu Glu Arg Leu Tyr
385 390 395 400

```
Gln Leu Gln Phe Asp Leu Ile Val Lys Asp Lys Val Pro Ile Ala Glu
                405                 410                 415

Ile Leu Phe His Pro Gly Gly Gly Asn Ala Val Ser Ser Glu Phe Trp
            420                 425                 430

Gly Leu Leu Pro Phe Ala Arg Gly Asn Ile His Ile Ser Ser Asn Asp
        435                 440                 445

Pro Thr Ala Pro Ala Ala Ile Asn Pro Asn Tyr Phe Met Phe Glu Trp
    450                 455                 460

Asp Gly Lys Ser Gln Ala Gly Ile Ala Lys Tyr Ile Arg Lys Ile Leu
465                 470                 475                 480

Arg Ser Ala Pro Leu Asn Lys Leu Ile Ala Lys Glu Thr Lys Pro Gly
                485                 490                 495

Leu Ser Glu Ile Pro Ala Thr Ala Ala Asp Glu Lys Trp Val Glu Trp
            500                 505                 510

Leu Lys Ala Asn Tyr Arg Ser Asn Phe His Pro Val Gly Thr Ala Ala
        515                 520                 525

Met Met Pro Arg Ser Ile Gly Gly Val Val Asp Asn Arg Leu Arg Val
    530                 535                 540

Tyr Gly Thr Ser Asn Val Arg Val Val Asp Ala Ser Val Leu Pro Phe
545                 550                 555                 560

Gln Val Cys Gly His Leu Val Ser Thr Leu Tyr Ala Val Ala Glu Arg
                565                 570                 575

Ala Ser Asp Leu Ile Lys Glu Asp Ala Lys Ser Ala
            580                 585
```

<210> 2
<211> 3226
<212> DNA
<213> Aspergillus oryzae

<400> 2

```
taggttttgt cacagccagg gtccgcgaaa accttactag cataaatcaa ccaccaaaca      60
cagtattcgg accgggtcta gttgcgatat cttcgttaac ggaatctgga ctctggtgta     120
atttcggagt ggacgtcggg agaatgatcc ttgggcttca tagcgcggcg atcaggtcaa     180
```

```
tgaggcaatt ttaggcctat tggagacttc ggatcctaga cattctgccg agccactttg    240
cctagactat tcctgacggg tcgggacttc accggtgaat gaattactcg gtatgccgat    300
tctgaataat accttaataa tgggaatttg atccactttt gggttcttcg ctaccacaga    360
gatgcgcact aaagattatt tgggcttagc tcgaatgtcc ctaacagtag cgtgtattgc    420
atcgtgctcg ctatagattg cagaaccata atctgagttt gagaacgtgt caactctcct    480
attaatcaat gaaagggaaa ggatgtgata tctaggagtg gaagattgag ctagcataga    540
taatatgggt ttaatcttcg tatgagatag agtaatagac ctttcgctgc tagagagcat    600
accacgcaaa gataatgcaa cgaagggacc ctcaactcga tcttatcaga tcagaaacgt    660
ccaccagttg cggcagaatt ccgcacagca gtattcgatt gatggaccca ctagcatgag    720
ctgataaatc catatctgct gaaacattag cttgcttaga agttgcttat ggatcattag    780
cttcattaac tggaggcagg atttgcagag aacttcacta tgatgcaatt caatcaccgt    840
gccgatagta cccagcagta aggtgaacgg aacccttcga attagactaa aatcaaaaaa    900
tatcatgcag tcatggactg catatataat ggttgatgcc agccttttg cgaaaggatc     960
ttcttcaagg cggacagaga tcgacgcttg acatccaaac atgctcttct cactggcatt   1020
cctgagtgcc ctgtcgctgg ccacggcatc accggctgga cgggccaaga acactacgac   1080
atacgactac atcgttgtgg gaggcggcac aagtggtctt gtggtcgcaa atcgcctttc   1140
tgagaacccc gatgtctccg ttcttctgct tgaggccggt gcttctgtgt tcaacaaccc   1200
ggacgtaacc aacgctaacg gttatggatt ggcctttggc tcggccatcg actggcagta   1260
ccagtctatt aaccaaagct atgcaggagg taaacagcaa gttctgcgtg ctggtaaggc   1320
ccttggagga accagtacaa tcaatggtat gcttctatgg atgatctctt agtcggcatg   1380
gaccactgac gaccacagga atggcctata cccgcgcaga ggatgtccag attgacgttt   1440
ggcagaaact tggaaacgaa ggttggacgt ggaaagatct cctaccatac tacctgaaga   1500
gtgaaaactt gacggcccct accagctctc aggttgctgc tggcgctgct tataaccctg   1560
ccgtgaatgg aaaagaaggt cctctcaagg tcggctggtc gggaagcctg gcctccggta   1620
atctgtcagt tgctctgaac cgtacgttcc aagccatgga ggatgtcaat ggaggcaaga   1680
tgcgtggctt caacatctac ccatccaccc tcgacgttga cctcaatgtc cgcgaagatg   1740
cagcccgggc atactacttc ccttatgatg acaggaagaa ccttcacctg ctggagaaca   1800
ccactgccaa ccgccttttc tggaagaacg gctctgctga ggaagctatt gcggatggtg   1860
tcgagatcac ctccgctgat ggcaaggtca ctcgtgtgca tgcaaagaaa gaggtcatca   1920
tctctgctgg tgccctgcgg tctcctctca ttctcgagct ttcaggagtt ggaaacccaa   1980
```

cgtaagtgtt ccactgatgc cagcccctct ctatcaccgt ctctgaccct cgtagcatcc 2040

tcaaaaagaa caacataacc ccacgtgtcg atctccccac cgttggggag aacctccaag 2100

accagttcaa caacggcatg gctggcgaag gatacggcgt ccttgccggt gcctcaaccg 2160

tgacctaccc ttccatctcc gacgtcttcg gtaacgagac tgactctatc gttgcatctc 2220

tccgatctca actctccgac tacgccgccg cgaccgtcaa ggtcagcaac ggccacatga 2280

agcaggagga ccttgagcgc ctctaccagc tccaatttga cctcatcgtc aaggacaagg 2340

tccctatcgc cgagatcctc ttccaccccg gtggtggaaa cgccgtgtcc tccgaattct 2400

ggggcttgct tcccttcgcc cgtggcaaca tccacattag ctccaatgac ccgactgctc 2460

ccgccgccat caaccctaac tactttatgt tcgaatggga cggcaagagc caggccggta 2520

tcgccaagta catcaggaag attctccgca gcgcaccatt gaacaaactt attgcgaagg 2580

aaaccaagcc cggtctctct gagattccgg ccactgctgc ggatgagaag tgggttgaat 2640

ggctcaaggc taactgtaag ttgaatcctt tcttggcttc gatggtgagt ctgacgtgag 2700

ctctctagat cgttccaact tccaccccgt cggaactgct gccatgatgc ctcgttccat 2760

tggtggcgtt gttgataacc gtctccgggt ctatggtacc agcaatgttc gcgtcgtaga 2820

tgcgtctgtc ctgcccttcc aggtttgcgg ccacttggtt agcacgcttt atgccgttgc 2880

cgagcgcgct tccgacttga ttaaggagga tgcgaagagt gcttagtttg gtagataaag 2940

ctgcattatc gtagggacag tgtcccgtac caattcgtgc accgtatgaa gagagaaagt 3000

ggtttcagaa ttgggttgtc aaatgtggat ttctgtttca ttgtttaatg tattattagt 3060

ctggattttg tggctgtttg aatgcagtga ctcttggtta cataatttga tacggggtat 3120

gttcgcttgt tcagtcatcg cacgtgtaat gtatggcata cctaggttat tggtaactca 3180

tattacaaaa ttgaagagaa cgagagatcg aagtatttcc catggc 3226

<210> 3
<211> 25
<212> DNA
<213> Artificial

<220>
<223> primer FG-F

<400> 3
taggttttgt cacagccagg gtccg 25

<210> 4
<211> 25
<212> DNA
<213> Artificial

<220>

<223> primer FG-R

<400> 4
gccatgggaa atacttcgat ctctc 25

<210> 5
<211> 18
<212> DNA
<213> Artificial

<220>
<223> primer FG-1

<400> 5
agtgaaaact tgacggcc 18

<210> 6
<211> 18
<212> DNA
<213> Artificial

<220>
<223> primer FG-2

<400> 6
ttcaggagtt ggaaaccc 18

<210> 7
<211> 18
<212> DNA
<213> Artificial

<220>
<223> primer FG-3

<400> 7
tatgttcgaa tgggacgg 18

<210> 8
<211> 18
<212> DNA
<213> Artificial

<220>
<223> primer FG-4

<400> 8
agaacgtgtc aactctcc 18

<210> 9
<211> 18
<212> DNA
<213> Artificial

<220>
<223> primer FG-5

<400> 9

tatgcatttc tcactggc 18

<210> 10
<211> 18
<212> DNA
<213> Artificial

<220>
<223> primer FG-6

<400> 10
gattgtactg gttcctcc 18

<210> 11
<211> 18
<212> DNA
<213> Artificial

<220>
<223> primer FG-7

<400> 11
tgcaccgtat gaagagag 18

<210> 12
<211> 18
<212> DNA
<213> Artificial

<220>
<223> primer FG-8

<400> 12
ttgctggtac catagacc 18

<210> 13
<211> 18
<212> DNA
<213> Artificial

<220>
<223> primer FG-9

<400> 13
ttgctgacct tgacggtc 18

<210> 14
<211> 18
<212> DNA
<213> Artificial

<220>
<223> primer FG-10

<400> 14
attgaggtca acgtcgag 18

<210> 15

<211> 18
<212> DNA
<213> Artificial

<220>
<223> primer FG-11

<400> 15
gcacggtgat tgaattgc 18

<210> 16
<211> 18
<212> DNA
<213> Artificial

<220>
<223> primer FG-12

<400> 16
tctgtggtag cgaagaac 18

<210> 17
<211> 18
<212> DNA
<213> Artificial

<220>
<223> primer M13-M5

<400> 17
cgaaaggggg atgtgctg 18

<210> 18
<211> 18
<212> DNA
<213> Artificial

<220>
<223> primer M13-RV2

<400> 18
cttccggctc gtatgttg 18

<210> 19
<211> 3241
<212> DNA
<213> Aspergillus oryzae

<400> 19

taggttttgt cacagccagg gtccgcgaaa accttactag cataaatcaa ccaccaaaca 60

cagtattcgg accgggtcta gttgcgacat cttcgttaac ggaatctgga ctctggtgta 120

```
atttcggagt ggacgtcggg agaatgatcc ttgggtttca tagcgcggcg atcaggtcaa    180
tgaggcaatt ttaggcctat tggagacttc ggatcctaga cattctgccg agccactttg    240
cctagactat tcctgacggg tcgggacttc accggtgaat gaattactcg gtatgccgat    300
tctgaataat accttaataa tgggaatttg atccactttt gggttcttcg ctaccacaga    360
gatgcgcact aaagattatt tgggcttagc tcgaatgtcc ctaacagtag cgtgtattgc    420
atcgtgctcg ctatagattg cagaaccata atctgagttt gagaacgtgt caactctcct    480
attaatcaat gaaagggaaa ggatgtgata tctaggagtg gaagattgag ctagcataga    540
taatatgggt ttaatcttcg tatgagatag agtaatagac ctttcgctgc tagagagcat    600
accacgcaaa gataatgcaa cgaagggacc ctcaactcga tcttatcaga tcagaaacgt    660
ccaccagttg cggcagaatt ccgcacagca gtattcgatt gatggaccca ctagcatgag    720
ctgataaatc catatctgct gaaacattag cttgcttaga agttgcttat ggatcattag    780
cttcattaac tggaggcagg atttgcagag aacttcacta tgatgcaatt caatcaccgt    840
gccgatagta cccagcagta aggtgaacgg aacccttcga attagactaa aatcaaaaaa    900
tatcatgcag tcatggactg catatataat ggttgatgcc agcctttttg cgaaaggatc    960
ttcttcaagg cggacagaga tcgacgcttg acatccaaac atgctcttct cactggcatt   1020
cctgagtgcc ctgtcgctgg ccacggcatc accggctgga cgggccaaga acactacgac   1080
atacgactac atcgttgtgg gaggcggcac aagtggtctt gtggtcgcaa atcgcctttc   1140
tgagaacccc gatgtctccg ttcttctgct tgaggccggt gcttctgtgt tcaacaaccc   1200
ggacgtaacc aacgctaacg gttatggatt ggcctttggc tcggccatcg actggcagta   1260
ccagtctatt aaccaaagct atgcaggagg taaacagcaa gttctgcgtg ctggtaaggc   1320
ccttggagga accagtacaa tcaatggtat gcttctatgg atgatctctt agtcggcatg   1380
gaccactgac gaccacagga atggcctata cccgcgcaga ggatgtccag attgacgttt   1440
ggcagaaact tggaaacgaa ggttggacgt ggaaagatct cctaccatac tacctgaaga   1500
gtgaaaactt gacggcccct accagctctc aggttgctgc tggcgctgct tataaccctg   1560
ccgtgaatgg aaaagaaggt cctctcaagg tcggctggtc gggaagcctg gcctccggta   1620
atctgtcagt tgctctgaac cgtacgttcc aagccgctgg tgttccatgg gttgaggatg   1680
tcaatggagg caagatgcgt ggcttcaaca tctacccatc caccctcgac gttgacctca   1740
atgtccgcga agatgcagcc cgggcatact acttccctta tgatgacagg aagaaccttc   1800
acctgctgga gaacaccact gccaaccgcc ttttctggaa gaacggctct gctgaggaag   1860
ctattgcgga tggtgtcgag atcacctccg ctgatggcaa ggtcactcgt gtgcatgcaa   1920
```

agaaagaggt catcatctct gctggtgccc tgcggtctcc tctcattctc gagctttcag 1980

gagttggaaa cccaacgtaa gtgttccact gatgccagcc cctctctatc accgtctctg 2040

accctcgtag catcctcaaa aagaacaaca taaccccacg tgtcgatctc cccaccgttg 2100

gggagaacct ccaagaccag ttcaacaacg gcatggctgg cgaaggatac ggcgtccttg 2160

ccggtgcctc aaccgtgacc tacccttcca tctccgacgt cttcggtaac gagactgact 2220

ctatcgttgc atctctccga tctcaactct ccgactacgc cgccgcgacc gtcaaggtca 2280

gcaacggcca catgaagcag gaggaccttg agcgcctcta ccagctccaa tttgacctca 2340

tcgtcaagga caaggtccct atcgccgaga tcctcttcca ccccggtggt ggaaacgccg 2400

tgtcctccga attctggggc ttgcttccct tcgcccgtgg caacatccac attagctcca 2460

atgacccgac tgctcccgcc gccatcaacc ctaactactt tatgttcgaa tgggacggca 2520

agagccaggc cggtatcgcc aagtacatca ggaagattct ccgcagcgca ccattgaaca 2580

aacttattgc gaaggaaacc aagcccggtc tctctgagat tccggccact gctgcggatg 2640

agaagtgggt tgaatggctc aaggctaact gtaagttgaa tcctttcttg gcttcgatgg 2700

tgagtctgac gtgagctctc tagatcgttc caacttccac cccgtcggaa ctgctgccat 2760

gatgcctcgt tccattggtg gcgttgttga taaccgtctc cgggtctatg gtaccagcaa 2820

tgttcgcgtc gtagatgcgt ctgtcctgcc cttccaggtt tgcggccact tggttagcac 2880

gctttatgcc gttgccgagc gcgcttccga cttgattaag gaggatgcga agagtgctta 2940

gtttggtaga taaagctgca ttatcgtagg gacagtgtcc cgtaccaatt cgtgcaccgt 3000

atgaagagag aaagtggttt cagaattggg ttgtcaaatg tggatttctg tttcattgtt 3060

taatgtatta ttagtctgga ttttgtggct gtttgaatgc agtgactctt ggttacataa 3120

tttgatacgg ggtatgttcg cttgttcagt catcgcacgt gtaatgtatg gcatacctag 3180

gttattggta actcatatta caaaattgaa gagaacgaga gatcgaagta tttcccatgg 3240

c 3241

<210> 20
<211> 593
<212> PRT
<213> Aspergillus oryzae

<400> 20

```
Met Leu Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val
            20                  25                  30
```

Val Gly Gly Gly Thr Ser Gly Leu Val Val Ala Asn Arg Leu Ser Glu
35 40 45

Asn Pro Asp Val Ser Val Leu Leu Leu Glu Ala Gly Ala Ser Val Phe
50 55 60

Asn Asn Pro Asp Val Thr Asn Ala Asn Gly Tyr Gly Leu Ala Phe Gly
65 70 75 80

Ser Ala Ile Asp Trp Gln Tyr Gln Ser Ile Asn Gln Ser Tyr Ala Gly
85 90 95

Gly Lys Gln Gln Val Leu Arg Ala Gly Lys Ala Leu Gly Gly Thr Ser
100 105 110

Thr Ile Asn Gly Met Ala Tyr Thr Arg Ala Glu Asp Val Gln Ile Asp
115 120 125

Val Trp Gln Lys Leu Gly Asn Glu Gly Trp Thr Trp Lys Asp Leu Leu
130 135 140

Pro Tyr Tyr Leu Lys Ser Glu Asn Leu Thr Ala Pro Thr Ser Ser Gln
145 150 155 160

Val Ala Ala Gly Ala Ala Tyr Asn Pro Ala Val Asn Gly Lys Glu Gly
165 170 175

Pro Leu Lys Val Gly Trp Ser Gly Ser Leu Ala Ser Gly Asn Leu Ser
180 185 190

Val Ala Leu Asn Arg Thr Phe Gln Ala Ala Gly Val Pro Trp Val Glu
195 200 205

Asp Val Asn Gly Gly Lys Met Arg Gly Phe Asn Ile Tyr Pro Ser Thr
210 215 220

Leu Asp Val Asp Leu Asn Val Arg Glu Asp Ala Ala Arg Ala Tyr Tyr
225 230 235 240

Phe Pro Tyr Asp Asp Arg Lys Asn Leu His Leu Leu Glu Asn Thr Thr
245 250 255

Ala Asn Arg Leu Phe Trp Lys Asn Gly Ser Ala Glu Glu Ala Ile Ala
260 265 270

```
Asp Gly Val Glu Ile Thr Ser Ala Asp Gly Lys Val Thr Arg Val His
        275                 280                 285

Ala Lys Lys Glu Val Ile Ile Ser Ala Gly Ala Leu Arg Ser Pro Leu
    290                 295                 300

Ile Leu Glu Leu Ser Gly Val Gly Asn Pro Thr Ile Leu Lys Lys Asn
305                 310                 315                 320

Asn Ile Thr Pro Arg Val Asp Leu Pro Thr Val Gly Glu Asn Leu Gln
                325                 330                 335

Asp Gln Phe Asn Asn Gly Met Ala Gly Glu Gly Tyr Gly Val Leu Ala
            340                 345                 350

Gly Ala Ser Thr Val Thr Tyr Pro Ser Ile Ser Asp Val Phe Gly Asn
        355                 360                 365

Glu Thr Asp Ser Ile Val Ala Ser Leu Arg Ser Gln Leu Ser Asp Tyr
    370                 375                 380

Ala Ala Ala Thr Val Lys Val Ser Asn Gly His Met Lys Gln Glu Asp
385                 390                 395                 400

Leu Glu Arg Leu Tyr Gln Leu Gln Phe Asp Leu Ile Val Lys Asp Lys
                405                 410                 415

Val Pro Ile Ala Glu Ile Leu Phe His Pro Gly Gly Gly Asn Ala Val
            420                 425                 430

Ser Ser Glu Phe Trp Gly Leu Leu Pro Phe Ala Arg Gly Asn Ile His
        435                 440                 445

Ile Ser Ser Asn Asp Pro Thr Ala Pro Ala Ala Ile Asn Pro Asn Tyr
    450                 455                 460

Phe Met Phe Glu Trp Asp Gly Lys Ser Gln Ala Gly Ile Ala Lys Tyr
465                 470                 475                 480

Ile Arg Lys Ile Leu Arg Ser Ala Pro Leu Asn Lys Leu Ile Ala Lys
                485                 490                 495

Glu Thr Lys Pro Gly Leu Ser Glu Ile Pro Ala Thr Ala Ala Asp Glu
            500                 505                 510

Lys Trp Val Glu Trp Leu Lys Ala Asn Tyr Arg Ser Asn Phe His Pro
```

515 520 525

Val Gly Thr Ala Ala Met Met Pro Arg Ser Ile Gly Gly Val Val Asp
530 535 540

Asn Arg Leu Arg Val Tyr Gly Thr Ser Asn Val Arg Val Val Asp Ala
545 550 555 560

Ser Val Leu Pro Phe Gln Val Cys Gly His Leu Val Ser Thr Leu Tyr
565 570 575

Ala Val Ala Glu Arg Ala Ser Asp Leu Ile Lys Glu Asp Ala Lys Ser
580 585 590

Ala